(19) Europäisches Patentamt
European Patent Office
Office européen des brevets

(11) **EP 4 441 237 B1**

(12) **EUROPÄISCHE PATENTSCHRIFT**

(45) Veröffentlichungstag und Bekanntmachung des Hinweises auf die Patenterteilung:
**26.03.2025 Patentblatt 2025/13**

(21) Anmeldenummer: **21816464.8**

(22) Anmeldetag: **29.11.2021**

(51) Internationale Patentklassifikation (IPC):
**C12P 13/00** (2006.01)  **C12P 11/00** (2006.01)
**C12P 13/04** (2006.01)  **C12N 9/88** (2006.01)

(52) Gemeinsame Patentklassifikation (CPC):
**C12P 11/00; C12N 9/88; C12P 13/001; C12P 13/04; C12Y 402/99**

(86) Internationale Anmeldenummer:
**PCT/EP2021/083369**

(87) Internationale Veröffentlichungsnummer:
**WO 2023/094010 (01.06.2023 Gazette 2023/22)**

(54) **VERFAHREN ZUR HERSTELLUNG VON TAURIN**

PROCESS FOR PRODUCING TAURINE

PROCÉDÉ POUR LA PRODUCTION DE TAURINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**09.10.2024 Patentblatt 2024/41**

(73) Patentinhaber: **Wacker Chemie AG**
**81671 München (DE)**

(72) Erfinder:
• **PFALLER, Rupert**
**80993 München (DE)**
• **WICH, Günter**
**81377 München (DE)**

(74) Vertreter: **Belz, Ferdinand et al**
**Wacker Chemie AG**
**Gisela-Stein-Straße 1**
**81671 München (DE)**

(56) Entgegenhaltungen:
**WO-A1-2017/083351    WO-A1-2023/094011**

• **ONO, K. ET AL.: "Synthesis of L-cysteine derivatives containing stable sulfur isotopes and application of this synthesis to reactive sulfur metabolome", FREE RADICAL BIOLOGY & MEDICINE, vol. 106, 9 February 2017 (2017-02-09), pages 69 - 79, XP029960555, DOI: 10.1016/J.FREERADBIOMED.2017.02.023**

Bemerkungen:
•Das gesamte Dokument mit Referenztabelle(n) und Sequenzliste(n) kann von der Webseite des EPA heruntergeladen werden
•Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Herstellung von Taurin aus O-Acetyl-L-Serin (OAS) mittels Biotransformation, wobei in einem 1. Verfahrensschritt (Biotransformation 1) L-Cysteinsäure aus O-Acetyl-L-Serin (OAS) mit einem Enzym ausgewählt aus der Klasse der OAS-Sulfhydrylasen (EC 4.2.99.8) in Gegenwart eines Salzes der schwefligen Säure hergestellt wird und anschließend in einem 2. Verfahrensschritt (Biotransformation 2) L-Cysteinsäure zu Taurin decarboxyliert wird, wobei im 1. Verfahrensschritt die Biotransformation unter aktiver pH-Kontrolle durchgeführt wird, die OAS-Konzentration im Ansatz mindestens 10 g/L beträgt und es sich bei der OAS-Sulfhydrylase um CysM handelt. Durch diese Biotransformation wird Taurin zur Verfügung gestellt.

Taurin (2-Aminoethansulfonsäure, CAS-Nummer 107-35-7) ist eine Aminosulfonsäure, die natürlicherweise in der Natur als Abbauprodukt der Aminosäuren Cystein und Methionin vorkommt. Taurin ist Bestandteil von Energiedrinks und wird auch in der Tiernahrung, z.B. für Katzen oder in der Fischzucht (Salze and Davis (2015) Aquaculture 437: 215-229), eingesetzt. Taurin werden jedoch auch gesundheitsfördernde Effekte zugeschrieben (Ripps and Shen (2012), Molecular Vision 18: 2673-2686).

In der Natur kommt Taurin fast ausschließlich im Tierreich vor, mit nur wenigen Beispielen für ein Vorkommen in Bakterien, Algen oder Pflanzen. Es gibt verschiedene biosynthetische Wege zum Taurin (für eine Übersicht siehe z.B. die KEGG Pathway Datenbank: "Taurine and hypotaurine metabolism"), u.a. ausgehend von L-Cystein. Die wichtigsten Syntheseschritte, die von L-Cystein zu Taurin führen, sind in den Gleichungen (1) bis (5) dargestellt.

$$\text{(1) L-Cystein} + O_2 \rightarrow \text{L-Cysteinsulfinsäure}$$

$$\text{(2) L-Cysteinsulfinsäure} + \tfrac{1}{2}\, O_2 \rightarrow \text{L-Cysteinsäure}$$

$$\text{(3) L-Cysteinsulfinsäure} \rightarrow \text{Hypotaurin} + CO_2$$

$$\text{(4) Hypotaurin} + \tfrac{1}{2}\, O_2 \rightarrow \text{Taurin}$$

$$\text{(5) L-Cysteinsäure} \rightarrow \text{Taurin} + CO_2$$

(1) In einem ersten Schritt wird L-Cystein durch das Enzym Cysteindioxygenase (CDO, EC 1.13.11.20) zu L-Cysteinsulfinsäure (3-Sulfinoalanin, CAS Nummer 207121-48-0) oxidiert.
(2) Cysteinsulfinatoxidase (bisher ein eher hypothetischer enzymatischer Schritt) oxidiert L-Cysteinsulfinsäure weiter zur L-Cysteinsäure ((R)-2-Amino-3-sulfopropionsäure, CAS Nummer 23537-25-9).
(3) Cysteinsulfinsäure Decarboxylase (CSAD, EC 4.1.1.29) decarboxyliert L-Cysteinsulfinsäure zu Hypotaurin (2-Aminoethansulfinsäure, CAS Nummer 300-84-5).
(4) Bisher nicht vollständig geklärt, wird Hypotaurin zu Taurin oxidiert.
(5) In einem Schritt ähnlich wie (3) wird L-Cysteinsäure durch geeignete CSAD-Enzyme zu Taurin decarboxyliert.

Für den kommerziellen Gebrauch wird Taurin z.Z. chemisch hergestellt. Bekannt ist z.B. ein Verfahren der Fa. Changshu Yudong Chemical Factory, das von Ethylen ausgeht und über Ethylenimin zum Taurin führt. Mit dem von Konsumentenwünschen beförderten Trend weg von chemisch hergestellten zu nachhaltig hergestellten Inhaltsstoffen werden zunehmend biotechnologische Verfahren zur Herstellung von Taurin untersucht. Der Stand der Technik stützt sich auf Metabolic Engineering Ansätze, bei denen geeignete Biosynthesegene heterolog in einem Produktionsstamm exprimiert werden und Taurin oder seinen biosynthetischen Vorläufer Hypotaurin produzieren.

Honjoh et al. (2010), Amino Acids 38: 173-1183 beschreiben einen gentechnisch veränderten Hefestamm, der die CDO- und CSAD-Gene aus Karpfen (*Cyprinus carpio*) heterolog exprimiert. Wurde der Anzucht des gentechnisch veränderten Stammes L-Cystein zugesetzt, dann konnte die Produktion von Hypotaurin als Hauptprodukt und daneben ein geringerer Anteil an Taurin beobachtet werden. Hypotaurin konnte durch Behandlung mit $H_2O_2$ zu Taurin oxidiert werden. Obwohl *S. cerevisiae* an sich in der Lage ist, Cystein aus seinem eigenen Stoffwechsel zu produzieren, war die externe Zugabe von L-Cystein in das Anzuchtmedium erforderlich, um Hypotaurin und Taurin zu produzieren.

Tevatia et al. (2019), Algal Research 40: 101491, setzen ebenfalls heterolog exprimierte CDO- und CSAD-Gene aus Karpfen zur Produktion von Taurin in der Alge *Chlamydomonas reinhardtii* ein. Die intrazelluläre Taurinausbeute betrug 0,14 mg Taurin/g Trockenbiomasse.

Joo et al. (2018), J. Agric. Food Chem. 66: 13454 - 13463, beschreiben einen gentechnisch veränderten Stamm des Bakteriums *Corynebakterium glutamicum* mit einer Produktionsleistung von 0,5 g/L Taurin in der Anzucht im Schüttelkolben. Dabei wurde Taurin offensichtlich intrazellulär angehäuft und nicht in das Anzuchtmedium sekretiert.

Zur Synthese von Taurin wurden in dem Stamm die Gene einer L-Cysteinsäuresynthase, einer Cystein Dioxygenase und einer L-Cysteinsulfinsäure Decarboxylase heterolog exprimiert. Weiterhin wurde ein Repressorgen der Methionin- und Cystein-Biosynthese inaktiviert, was gleichzeitig eine verbesserte Schwefelaufnahme bewirkte. Neben der geringen Ausbeute ist es im Hinblick auf die Produktisolierung als grundsätzlicher Nachteil anzusehen, dass Taurin intrazellulär produziert wird. Dies erfordert den aufwendigen Aufschluss der Zellen, um das Taurin für die weitere Verarbeitung freizusetzen.

WO17213142A1 (Ajinomoto) beschreibt einen Taurinproduzierenden Stamm, der durch heterologe Expression einer Cystein Dioxygenase und einer L-Cysteinsulfinsäure Decarboxylase in einem zur Cysteinproduktion befähigten Stamm erhalten wurde. Hauptprodukt war Hypotaurin mit max. 450 $\mu$M Ausbeute, das nur durch nachträgliche alkalische Behandlung und auch nur in geringer Ausbeute zu Taurin umgewandelt werden konnte.

Ono et al. (Free Radical Biology and Medicine 106, S. 69-79, 2017) offenbart, dass die OAS-Sulfhydrylasen CysM und CysK aus *Salmonella entericum* LT2 analog zu den Enzymen aus *E. coli* (s. Maier, Nature Biotechnology 21, S. 422-427, 2003, Tab. 1) in der Lage sind, in hoher Ausbeute von über 70% aus den Substraten OAS und Sulfid Cystein zu bilden. Ono offenbart darüber hinaus, dass CysM und CysK aus *Salmonella entericum* LT2 zudem aus OAS in Gegenwart von $Na_2SO_3$ unter ansonsten gleichen Reaktionsbedingungen in geringen Mengen, d.h. in einer molaren Ausbeute von unter 0,2%, das Cystein-Derivat L-Cysteinsäure (Cysteinsulfonat, Salz der Cysteinsäure) bilden können.

US9267148B2, US2012/0222148A1, US2018/0028474A1, US2019/0085339, WO2017/176277A1 und WO2019/094051 (Plant Sensory Systems) beschreiben Taurin-produzierende Stämme und Organismen, die in verschiedenen Formen die heterologe Expression einer Cystein Dioxygenase und einer L-Cysteinsulfinsäure Decarboxylase umfassen. Die Anmeldungen zielen hauptsächlich auf die Produktion von Taurin in Pflanzen ab, wobei keine Aussagen über Ausbeuten gemacht werden.

US20190062757A1 (KnipBio) beschreibt heterologe Produktionsstämme zur Herstellung von Taurin oder seiner Vorläufersubstanzen.

Der Stand der Technik offenbart somit verschiedene Metabolic Engineering-Ansätze, um Hypotaurin, bzw. Taurin in heterologen Produktionssystemen herzustellen. Sofern die Metabolic Engineering-Ansätze Produktausbeuten offenbaren, sind diese für den technischen Einsatz zu gering.

Der Stand der Technik stellt weiterhin Verfahren zur Herstellung nicht-proteinogener (unnatürlicher) Aminosäuren bereit, z.B. durch direkte Fermentation von Mikroorganismen (EP 1 191 106 B1, Wacker) oder durch O-Acetyl-L-Serin-Sulfhydrylase (OAS-Sulfhydrylase) katalysierte Biotransformation von OAS (EP 1 247 869 B1, Wacker). Die Verfahren beruhen darauf, dass eine OAS-Sulfhydrylase die Reaktion von OAS mit einem Nucleophil zu einer nicht-proteinogenen Aminosäure katalysiert nach der allgemeinen Gleichung (6):

$$\text{(6) OAS + Nucleophil -> unnat. Aminosäure + Acetat}$$

In EP 1 247 869 B1 (Wacker) wurde eine Vielzahl unterschiedlicher Nucleophile auf ihre Eignung als Nucleophil für die durch eine OAS-Sulfhydrylase katalysierte Reaktion mit OAS untersucht, darunter Selenide, Selenol, Azide, Cyanide, Azole und Isoxazolinone. Außerdem wurden auch Schwefelverbindungen aus der Gruppe der Thiosulfate und Thiole der allg. Formel H-S-R verwendet, wobei der Rest R ein einwertiger substituierter oder nicht substituierter Alkyl-, Alkoxy-, Aryl- oder Heteroarylrest war.

Diese Verfahren sind nicht zur Herstellung von L-Cysteinsäure und/oder Taurin geeignet.

Aufgabe der vorliegenden Erfindung war es, unter Umgehung eines durch Metabolic Engineering erzeugten heterologen Produktionsstammes ein biotechnologisches Verfahren zur Herstellung von Taurin durch Biotransformationen bereitzustellen.

Gelöst wird die Aufgabe durch ein Verfahren zur Herstellung von Taurin aus O-Acetyl-L-Serin (OAS) mittels Biotransformation, wobei in einem 1. Verfahrensschritt (Biotransformation 1) L-Cysteinsäure aus O-Acetyl-L-Serin (OAS) mit einem Enzym ausgewählt aus der Klasse der OAS-Sulfhydrylasen (EC 4.2.99.8) in Gegenwart eines Salzes der schwefligen Säure hergestellt wird und anschließend in einem 2. Verfahrensschritt (Biotransformation 2) L-Cysteinsäure zu Taurin decarboxyliert wird, wobei im 1. Verfahrensschritt die Biotransformation unter aktiver pH-Kontrolle durchgeführt wird, die OAS-Konzentration im Ansatz mindestens 10 g/L beträgt und es sich bei der OAS-Sulfhydrylase um CysM handelt.

Wie in der begleitenden Anmeldung EP 4 441 239 A1 und den Beispielen der vorliegenden Erfindung offenbart, wurde überraschend gefunden, dass sich Salze der schwefligen Säure (im Folgenden als Sulfite bzw. $SO_3^{2-}$ bezeichnet) als Nucleophil in Reaktion (6) eignen und in Erweiterung von EP 1 247 869 B1 (Wacker) in einer Reaktion entsprechend Gleichung (7) die Synthese der nicht-proteinogenen Aminosäure L-Cysteinsäure ermöglichen (Biotransformation 1).

$$\text{(7) OAS + } SO_3^{2-} \text{ -> L-Cysteinsäure + Acetat}$$

Die in der Biotransformation 1 hergestellte L-Cysteinsäure konnte ebenso unerwartet ohne weitere Aufarbeitung entsprechend Gleichung (5) durch ein rekombinant hergestelltes CSAD-Enzym zu Taurin decarboxyliert werden. (Biotransformation 2).

Der Vorteil des erfindungsgemäßen Verfahrens zur Herstellung von Taurin ist, dass es sich um ein nachhaltiges und technisch umsetzbares wirtschaftliches Biotransformationsverfahren zur Herstellung von Taurin handelt. Auf umweltgefährdende Chemikalien kann verzichtet werden. Es werden keine Rohstoffe fossiler Herkunft verbraucht und es fallen keine giftigen chemischen Abfälle und/oder Abgase an. Das Herstellverfahren der vorliegenden Erfindung ist daher umweltschonend und nachhaltig. Zudem verlangt das Verfahren weder extreme Reaktionsbedingungen noch spezielle Anlagen und ist daher leicht technisch umsetzbar. Solche Verfahren werden verstärkt nachgefragt.

Taurin aus dem erfindungsgemäßen Verfahren kann entweder ohne weitere Aufarbeitungsschritte direkt weiterverwendet oder aber mittels bekannter Methoden angereichert werden.

Im Rahmen der vorliegenden Erfindung werden Herstellverfahren wie folgt unterschieden:

1. Chemische Verfahren
2. Biotechnologische Verfahren:

a) durch *metabolic engineering*
*Metabolic Engineering* (auch *"Pathwaydesign"* genannt) ist im Gegensatz zur Biotransformation eine Methode der Biotechnologie, bei der Stoffwechselwege eines Organismus durch Optimierung, bzw. Veränderung genetischer und regulatorischer Prozesse verändert werden. Dabei können durch Ergänzung des Genoms mit Genen von Enzymen neue oder veränderte Enzyme in einen Organismus eingeführt werden, bzw. Gene endogener Enzyme verstärkt oder abgeschwächt exprimiert werden und dadurch neue Stoffwechselwege in einem Organismus etabliert oder bestehende Stoffwechselwege verstärkt oder abgeschwächt werden. Ziel des *Metabolic Engineering* ist, dass der Organismus ein Stoffwechselprodukt entweder neu oder ein zelleigenes Stoffwechselprodukt mit erhöhter Ausbeute produziert. In ein *Metabolic Engineering-Verfahren* werden keine für das Stoffwechselprodukt spezifischen Ausgangsstoffe wie ein Enzymsubstrat, wie z.B. OAS in der vorliegenden Erfindung, eingesetzt, sondern lediglich ein Nährstoffmedium, auch bezeichnet als Anzuchtmedium, welches für das Wachstum des betreffenden Organismus erforderlich ist und zusammengesetzt ist aus einer C-Quelle (z.B. Glucose), einer N-Quelle (z.B. ein Ammoniumsalz oder ein komplexes Aminosäuregemisch wie z.B. Pepton oder Hefeextrakt) und weiteren für das Wachstum erforderlichen Salzen. Solche Nährstoffmedien sind dem Fachmann aus der mikrobiologischen Praxis bekannt.
b) durch Biotransformation
Biotransformation ist definiert als Überführung eines oder mehrerer Edukte in ein Produkt unter enzymatischer Katalyse, wobei das Enzymsubstrat mit dem Enzym in einen Reaktionsansatz gegeben wird. Im Reaktionsansatz wird das zugesetzte Enzymsubstrat, wie in der vorliegenden Erfindung OAS, bzw. L-Cysteinsäure, enzymatisch umgesetzt. Dies geschieht in der vorliegenden Erfindung für OAS durch ein CysM Enzym aus der Klasse der OAS-Sulfhydrylasen (EC 4.2.99.8) in Gegenwart eines Salzes der schwefligen Säure, entsprechend Gleichung (7) und für L-Cysteinsäure durch ein Enzym ausgewählt aus der Klasse der Cysteinsulfinsäure-Decarboxylasen (CSAD, EC 4.1.1.29), entsprechend Gleichung (5). Das oder die Edukte können dabei aus chemischer oder biotechnologischer Herstellung stammen. Das im erfindungsgemäßen Verfahren eingesetzte OAS kann z.B. aus chemischer Synthese oder aus biotechnologischer Produktion durch Fermentation eines Produktionsstammes stammen. Die im Verfahren eingesetzte L-Cysteinsäure kann z.B. aus chemischer Synthese oder aus biotechnologischer Produktion durch Biotransformation von OAS stammen. Das oder die für die enzymatische Katalyse eingesetzten Enzyme stammen dabei entweder aus biotechnologischer Herstellung durch Anzucht von Produktionsstämmen, z.B. durch Fermentation oder es wird biologisches Material verwendet, welches das oder die Enzyme enthält (z.B. Pflanzen, Pilze, Algen, tierische Organe). Dabei kann die Biomasse aus der Anzucht des Produktionsstammes, bzw. das biologische Material direkt verwendet werden oder das Enzym wird je nach Erfordernissen der Biotransformation daraus isoliert. Die im erfindungsgemäßen Verfahren eingesetzten Enzyme CysM und CSADcc stammen aus biotechnologischer Herstellung durch Fermentation eines Produktionsstammes.

Im Rahmen der vorliegenden Erfindung ist ein Reaktionsansatz definiert als eine Mischung aus Edukt (Ausgangsstoff), Enzym und ggf. weiteren Reaktanden, bei dem das Edukt in ein Produkt überführt wird.

Die Ausbeute der Reaktion im Sinne der Erfindung ist definiert als die Menge des eingesetzten Edukts, welches unter Reaktionsbedingungen zum Produkt umgewandelt wird. Die Ausbeute kann angegeben werden in absoluter Menge (g oder mmol), als Volumenausbeute in absoluter, auf das Volumen bezogene Menge Produkt (mM oder g/L) oder als relative Ausbeute von Produkt in Prozent des eingesetzten Edukts (unter Berücksichtigung der Molekulargewichte des Edukts und des Produkts), auch bezeichnet als prozentuale Ausbeute. Fermentation ist ein Verfahrensschritt zur Herstellung (Kultivierung) von Zellkulturen im technischen Maßstab, bei dem ein bevorzugt mikrobieller Produktionsstamm unter definierten Bedingungen von Kulturmedium, Temperatur, pH, Sauerstoffzufuhr und Mediumdurchmischung zum Wachstum gebracht wird. Ziel der Fermentation ist, abhängig von der Konfiguration (genetischen Ausstattung) des Produktionsstammes, die Produktion eines Proteins/Enzyms oder eines Stoffwechselprodukts, jeweils mit möglichst hoher Ausbeute für die weitere Verwendung. Die Komponenten des erfindungsgemäßen Verfahrens, OAS, OAS-Sulfhydrylase CysM und Cysteinsulfinsäure Decarboxylase können durch Fermentation hergestellt werden. Endprodukt der Fermentation ist eine Fermenterbrühe, bestehend aus der Biomasse der Zellen des Produktionsstammes (Fermenterzellen) und dem von der Biomasse befreiten Fermentationsmedium (Fermentationsüberstand), das sich im Verlauf der Fermentation aus dem Anzuchtmedium und den von den Fermenterzellen sekretierten Stoffwechselprodukten gebildet hat. Die Zielprodukte der Fermentation können sich in den Fermenterzellen oder im Fermentationsmedium befinden. So findet sich OAS im Fermentationsmedium wieder, während die Enzyme OAS-Sulfhydrylase CysM und CSADcc sich in den

Fermenterzellen wiederfinden.

Als offener Leserahmen (open reading frame, ORF, gleichbedeutend mit cds, coding sequence) wird derjenige Bereich der DNS bzw. RNS bezeichnet, der mit einem Startcodon beginnt und mit einem Stopcodon endet und für die Aminosäuresequenz eines Proteins codiert. Der ORF wird auch als codierende Region oder Strukturgen bezeichnet.

Als Gen wird der DNS-Abschnitt bezeichnet, der alle Grundinformationen zur Herstellung einer biologisch aktiven RNS enthält. Ein Gen enthält den DNS-Abschnitt, von dem durch Transkription eine einzelsträngige RNS-Kopie hergestellt wird und die Expressionssignale, die an der Regulation dieses Kopiervorgangs beteiligt sind. Zu den Expressionssignalen zählen z.B. mindestens ein Promotor, ein Transkriptions-, ein Translationsstart und eine Ribosomenbindestelle (RBS). Des Weiteren sind als Expressionssignale ein Terminator und ein oder mehrere Operatoren möglich.

Eine mRNS, auch messenger-RNS oder Boten-RNS genannt, ist eine einzelsträngige Ribonukleinsäure (RNS), welche die genetische Information für den Aufbau eines Proteins trägt. Mit einer mRNS steht die Bauanleitung für ein bestimmtes Protein in einer Zelle zur Verfügung. Das mRNS-Molekül trägt die zum Proteinaufbau notwendige Botschaft aus der Erbinformation (DNS) an die proteinaufbauenden Ribosomen. In einer Zelle wird es gebildet als Transkript eines zu einem Gen gehörenden Teilabschnitts der DNS. Die in der DNS gespeicherte genetische Information wird dabei nicht verändert.

Gene eukaryotischer Organismen sind überwiegend sog. Mosaikgene und enthalten im Gegensatz zu prokaryotischen Genen auch nichtcodierende Abschnitte, sog. Introns (engl. *intragenic regions*). Codierende Sequenzen, sog. Exons (engl. *expressed regions*) sind DNS-Abschnitte eines eukaryotischen Gens, die nach der Transkription in RNS von den Ribosomen in die Aminosäure-Sequenz eines Proteins übersetzt werden. Die Introns werden nach der Transkription der DNS zur RNS aus dem Primärtranskript gespleißt. Die von Introns befreite proteincodierende RNS wird als messenger-RNS (mRNS), auch als "reife" mRNS bezeichnet. Diese wird weiteren Modifikationen wie dem Capping und der Polyadenylierung unterzogen. Der codierende Bereich der reifen mRNS wird dann in die Proteinsequenz übersetzt. Soll ein eukaryotisches Gen mit Exon/Intronstruktur in prokaryotischen Organismen exprimiert werden, ist es notwendig, die Proteinsequenz oder den codierenden Bereich der reifen mRNS in Intron-freie DNS zurückzuübersetzen, da bei Prokaryoten die Prozessierung der Exon/Intronstruktur nicht stattfindet. Wenn im Rahmen dieser Erfindung von *aus der Proteinsequenz* oder von aus *der mRNS abgeleiteten Gensequenzen* die Rede ist, ist genau dieser Prozess der Rückübersetzung gemeint. Es ist bevorzugt, dass gleichzeitig mit der Rückübersetzung der Proteinsequenz oder der mRNS-Sequenz in DNS-Sequenz eine Sequenzoptimierung, d.h. Anpassung an die Codon-Nutzung des entsprechenden Prokaryoten erfolgt (Codon-Optimierung).

Als Genkonstrukt wird ein DNS-Molekül bezeichnet, bei dem ein Gen verknüpft ist mit weiteren genetischen Elementen (z.B. Promotor, Terminator, Selektionsmarker, Replikationsursprung). Ein Genkonstrukt im Rahmen der Erfindung ist ein zirkuläres DNS-Molekül und wird als Plasmid, Vektor, bzw. Expressionsvektor bezeichnet. Die genetischen Elemente des Genkonstrukts bewirken seine extrachromosomale Vererbung während des Zellwachstum sowie die Produktion des vom Gen codierten Proteins.

Die Abkürzung WT (Wt) bezeichnet den Wildtyp. Als Wildtyp-Gen wird die Form des Gens bezeichnet, die natürlicherweise durch die Evolution entstanden und im Wildtyp-Genom vorhanden ist. Die DNS-Sequenz von Wt-Genen ist in Datenbanken wie NCBI öffentlich zugänglich.

**Biotransformation 1:**

Voraussetzung für das erfindungsgemäße Verfahren zur Herstellung von Taurin ist die Verfügbarkeit von OAS. Denkbar sind chemische Verfahren zur Herstellung von OAS, z.B. durch Acetylierung von L-Serin, was aufgrund der hohen L-Serin-Preise teuer ist, oder auch die Herstellung des Racemats O-Acetyl-D/L-Serin, das direkt verwendet werden kann, bzw. aus dem Racemat wird vorher OAS gewonnen, z.B. durch Racematspaltung. Bei der direkten Acetylierung kann N-Acetyl-L-Serin (NAS) als Nebenprodukt gebildet werden, z.B. durch nicht selektive Acetylierung an der Hydroxy- oder Aminogruppe von L-Serin oder die bekannte Umlagerung von OAS zu NAS bei neutralen bis alkalischen pH-Werten (Tai et al. (1995), Biochemistry 34: 12311-12322), was die Ausbeuten erniedrigt oder das vorherige Einführen einer Schutzgruppe an der Aminogruppe von L-Serin erfordert. Aus diesem Grund ist die direkte Acetylierung von L-Serin für ein wirtschaftliches Verfahren nicht praktikabel.

Bekannt ist die biotechnologische Herstellung von OAS. Hierbei kommen Organismen zum Einsatz, die einen deregulierten Cystein-Stoffwechsel aufweisen und deshalb einen hohen Spiegel an OAS bereitstellen. Ein fermentatives Verfahren zur Herstellung von OAS ist in EP 1 233 067 B1 (Wacker) offenbart bzw. im Beispiel 1 der vorliegenden Erfindung beschrieben.

Bevorzugt ist die biotechnologische Herstellung von OAS, insbesondere bevorzugt unter Verwendung des *Escherichia coli (E. coli)* Stammes W3110/pACYC-cysEX-GAPDH-ORF306 (Beispiel 1), hinterlegt nach Budapester Vertrag bei der DSMZ Deutsche Sammlung für Mikroorganismen und Zellkulturen GmbH (Braunschweig) unter der Nummer DSM 13495.

Ein Vorteil der vorliegenden Erfindung ist es, dass eine OAShaltige Fermenterbrühe wie sie z.B. aus einer nach EP 1 233 067 durchgeführten Fermentation erhalten wird, als Fermentationsüberstand nach Abtrennen der partikulären Biomasse wie z.B. durch Zentrifugation ohne weitere Aufarbeitungs-, Reinigungs- oder Isolierungsschritte wie u.a. Extraktion, Adsorption, Ionenaustauscher-Chromatographie, Präzipitation oder Kristallisation als OAS-Quelle direkt im erfindungsgemäßen Verfahren eingesetzt werden kann. Diese Vorgehensweise ist besonders ökonomisch und vermeidet die Isolierung einer instabilen Verbindung.

Der Fachmann kann mittels Isotopenanalyse feststellen, ob ein Stoff wie beispielsweise OAS oder L-Cysteinsäure, die er in das Verfahren einsetzen will, aus chemischer oder fermentativer Herstellung stammt. Ein zur Unterscheidung geeignetes Verfahren der Isotopenanalyse ist z.B. in Sieper et al., Rapid Commun. Mass Spectrom. (2006) 20: 2521-2527 beschrieben und beruht auf der Bestimmung der Isotopenverhältnisse für z.B. C oder N, welche verschieden sind, je nachdem ob ein Produkt aus chemischer (Erdölbasierter) oder fermentativer (aus Pflanzen-basierten Rohstoffen) Herstellung stammt.

OAS-Sulfhydrylasen sind bisher aus verschiedenen Pflanzen und Mikroorganismen isoliert worden. In *E. coli* z.B. existieren zwei OAS-Sulfhydrylase-Enzyme, die als CysK und CysM bezeichnet werden. Die zugehörigen Gene sind ebenfalls bekannt und tragen die Bezeichnung cysK bzw. cysM. CysM OAS-Sulfhydrylasen im Sinne der vorliegenden Erfindung sind dadurch gekennzeichnet, dass sie die Synthese der proteinogenen Aminosäure L-Cystein aus OAS nach Gleichung (8) katalysieren können, wobei in diesem Fall Sulfid als Nucleophil dient.

$$(8)\ \ OAS\ +\ S^{2-}\ ->\ L\text{-}Cystein\ +\ Acetat$$

Obwohl beide Enzyme einen sehr ähnlichen Reaktionsmechanismus besitzen und an der Biosynthese von L-Cystein beteiligt sind, besitzt CysM im Gegensatz zu CysK ein variables Substratspektrum bezüglich des Nucleophils, das nach Gleichung (6) mit OAS reagieren kann. Von CysM ist z.B. bekannt, dass es im Gegensatz zu CysK in der Lage ist, eine Reaktion von OAS mit Thiosulfat zu S-Sulfocystein (CAS-Nummer 1637-71-4) zu katalysieren. Diese Reaktion spielt beim Wachstum der Bakterien mit Thiosulfat als einziger Schwefelquelle eine wichtige Rolle. Weiterhin ist aus EP 1 247 869 B1 (Wacker) die Verwendung von CysM zur Herstellung nicht-proteinogener Aminosäuren bekannt.

Bevorzugt ist das Verfahren dadurch gekennzeichnet, dass es sich bei der OAS-Sulfhydrylase CysM um ein bakterielles Enzym, besonders bevorzugt um CysM des Stammes *E. coli* handelt.

Das Verfahren ist bevorzugt dadurch gekennzeichnet, dass die OAS-Sulfhydrylase CysM aus fermentativer Herstellung stammt, besonders bevorzugt aus der Fermentation eines *E. coli* Stammes und insbesondere bevorzugt aus der Fermentation des Stammes *E. coli* DH5α/pFL145.

Eine Durchführung der fermentativen biotechnologischen Herstellung von CysM mit dem Stamm *E. coli* DH5α/pFL145 ist in Beispiel 2 offenbart. Der Produktionsstamm besteht aus einem Wirtsstamm, wie in diesem Fall *E. coli* DH5α und einem Genkonstrukt, das für die Expression der OAS-Sulfhydrylase geeignet ist, bevorzugt das Genkonstrukt pFL145. Wirtsstamm und Genkonstrukt sowie die Herstellung des Produktionsstamms sind in EP 1 247 869 B1 beschrieben. Der Produktionsstamm ist hinterlegt nach Budapester Vertrag bei der DSMZ Deutsche Sammlung für Mikroorganismen und Zellkulturen GmbH (Brauschweig) unter der Nummer DSM 14088.

Die durch Fermentation des Produktionsstammes gewonnene Fermenterbrühe besteht aus den, die OAS-Sulfhydrylase enthaltenden Fermenterzellen und dem Fermentationsmedium (Fermentationsüberstand). Im erfindungsgemäßen Verfahren kann entweder die nicht weiter aufgearbeitete Fermenterbrühe eingesetzt werden oder aber auch eine Suspension der Fermenterzellen (resuspendierte Fermenterzellen), z.B. in einem Puffer, nach deren Isolierung aus der Fermenterbrühe, z.B. durch Zentrifugation oder Filtration. Die Herstellung CysM-haltiger, resuspendierter Fermenterzellen des Stammes *E. coli* DH5α/pFL145 ist in Beispiel 2 beschrieben.

Denkbar ist weiterhin, dass die OAS-Sulfhydrylase in Form eines Zellhomogenats nach mechanischem Aufschluss der Fermenterzellen oder in Form chemisch permeabilisierter Zellen (z.B. durch Chloroform) eingesetzt wird oder aber auch als Zellextrakt nach Abtrennung partikulärer Bestandteile aus dem Zellhomogenat oder auch als z.B. chromatographisch gereinigtes Enzym. Bevorzugt ist die Verwendung der OAS-Sulfhydrylase als nicht weiter aufgearbeitete Fermenterbrühe, als Zellsuspension resuspendierter Fermenterzellen oder aber als Zellhomogenat nach mechanischem Aufschluss resuspendierter Fermenterzellen oder in Form chemisch permeabilisierter Zellen (z.B. durch Chloroform). Besonders bevorzugt ist die Verwendung der OAS-Sulfhydrylase in Form resuspendierter Fermenterzellen oder als Zellhomogenat. Insbesondere bevorzugt ist die Verwendung resuspendierter Fermenterzellen des OAS-Sulfhydrylase Produktionsstammes.

Im erfindungsgemäßen Verfahren reagiert OAS mit Sulfit unter Katalyse einer OAS-Sulfhydrylase CysM nach Gleichung (7) zu L-Cysteinsäure.

Schwefelige Säure bildet eine Vielzahl, in reversiblen Gleichgewichten gleichzeitig vorliegender chemischer Spezies, deren jeweilige Eignung als Nucleophil in der erfindungsgemäßen Biotransformation nicht vorhersehbar war. So ist es bekannt, dass schwefelige Säure ($H_2SO_3$) die wässrige Lösung von gasförmigen $SO_2$ ist und als zweiwertige Säure, abhängig vom pH der wässrigen Lösung, in unterschiedlichen Gleichgewichten vorliegt, deren Spezies auch unterschiedlich als Nucleophil geeignet sind. Die folgenden Gleichgewichte (9) bis (13) sind bekannt:

$$(9) \quad SO_2(gasförgmig) <-> SO_2(gelöst)$$

$$(10) \quad SO_2(gelöst) + H_2O <-> H_2SO_3$$

$$(11) \quad H_2SO_3 <-> HSO_3^- + H^+$$

$$(12) \quad HSO_3^- <-> SO_3^{2-} + H^+$$

$$(13) \quad 2\ HSO_3^- <-> S_2O_5^{2-} + H_2O$$

Schwefelige Säure und ihre Salze werden in der Lebensmittelindustrie als Konservierungsstoffe eingesetzt, da sie antimikrobielle Wirkung entfalten. Das bedeutet, dass schwefelige Säure und ihre Salze Mikroorganismen abtöten können, was auf die Inaktivierung der für die Lebensfähigkeit des Mikroorganismus notwendigen Enzyme zurückzuführen ist.

Daher würde der Fachmann erwarten, dass auch das CysM-Enzym bei Einsatz von schwefeliger Säure oder deren Salze inaktiviert wird und L-Cysteinsäure mit dem in EP 1 247 869 B1 offenbarten Verfahren nicht zugänglich ist.

Aus den genannten Gründen war es für den Fachmann überraschend, dass unter Einsatz von Sulfit und OAS in einer Biotransformation L-Cysteinsäure als Ausgangsverbindung für ein biotechnologisches Verfahren zur Herstellung von Taurin hergestellt werden kann.

Grundsätzlich sind alle denkbaren Salze der schwefligen Säure für die Reaktion geeignet. Bevorzugt ist das Verfahren dadurch gekennzeichnet, dass als Salz einer schwefligen Säure $Na_2SO_3$, $K_2SO_3$, $(NH_4)_2SO_3$, $NaHSO_3$ (bzw. dessen Anhydrid $Na_2S_2O_5$) oder $KHSO_3$ verwendet wird. Besonders bevorzugt wird als Salz einer schwefligen Säure $Na_2SO_3$, $NaHSO_3$ (bzw. dessen Anhydrid $Na_2S_2O_5$) oder $(NH_4)_2SO_3$ und insbesondere bevorzugt $Na_2SO_3$ oder $NaHSO_3$ (bzw. dessen Anhydrid $Na_2S_2O_5$) eingesetzt.

Denkbar ist die Verwendung von gasförmigem Schwefeldioxid, das Anhydrid der schwefligen Säure, das in den Reaktionsansatz eingebracht werden kann, wo es zur schwefligen Säure $H_2SO_3$ hydratisiert und je nach pH in einem Gleichgewicht mit den deprotonierten Formen $HSO_3^-$ und $SO_3^{2-}$ vorliegt.

Bevorzugt ist das Verfahren dadurch gekennzeichnet, dass die Konzentration des Salzes der schwefligen Säure mindestens in äquimolarer Konzentration zu OAS vorliegt. Besonders bevorzugt liegt das Salz der schwefligen Säure mindestens in 1,5-fach molarem Überschuss, insbesondere bevorzugt in mindestens fünffach molarem Überschuss zu OAS vor.

In einer besonders bevorzugten Ausführungsform ist das Verfahren zur Herstellung von Taurin dadurch gekennzeichnet, dass sowohl die OAS-Sulfhydrylase CysM als auch OAS durch Fermentation hergestellt werden.

OAS als Edukt des erfindungsgemäßen Biotransformationsverfahrens isomerisiert ab einem pH-Wert von ca. pH 7 zu N-Acetyl-L-Serin (NAS) und ist dann nicht mehr für die Reaktion mit Sulfit zu L-Cysteinsäure geeignet. Der Mechanismus der Reaktion wurde in Tai et al. (1995), Biochemistry 34: 12311-12322 untersucht und beruht auf einem intramolekularen, nukleophilen Angriff der deprotonierten Aminogruppe am Carbonyl-Kohlenstoff des Acyl-Rests. Diese Reaktion wird mit abnehmendem pH-Wert unterdrückt, so dass die Verbindung z.B. bei pH 4,0 stabil ist.

Das erfindungsgemäße Biotransformationsverfahren zeichnet sich somit bevorzugt dadurch aus, dass die Reaktion unter pH-Bedingungen durchgeführt wird, welche die Isomerisierung von OAS zu NAS minimieren.

Die Reaktionstemperatur von Biotransformation 1 wird bevorzugt zwischen 5°C und 70°C gewählt. Bevorzugt ist eine Reaktionstemperatur zwischen 10°C und 60°C, besonders bevorzugt zwischen 15°C und 50°C und insbesondere bevorzugt zwischen 20°C und 40°C.

Die OAS Konzentration im Ansatz beträgt mindestens 10 g/L und bevorzugt mindestens 40 g/L.

Bei der Biotransformation von OAS beträgt die molare Ausbeute an L-Cysteinsäure bezogen auf die molare Einsatzmenge OAS bevorzugt mindestens 60%, besonders bevorzugt mindestens 70% und insbesondere bevorzugt mindestens 80%.

In einer weiteren bevorzugten Ausführung des Biotransformationsverfahrens 1 wird das Substrat OAS aus einer Vorlage in einem sog. Zulaufverfahren in den Reaktionsansatz aus OAS-Sulfhydrylase CysM und Sulfit zudosiert (Beispiel 5). Dabei wird zur Vermeidung der Isomerisierung von OAS zu NAS in der das OAS enthaltenden Vorlage bevorzugt ein pH ≤6,5, besonders bevorzugt ein pH ≤6,0 und insbesondere bevorzugt ein pH ≤5,5 eingestellt. Gleichzeitig wird der pH im Reaktionsansatz so eingestellt, dass er die Reaktion zu L-Cysteinsäure begünstigt, bevorzugt pH ≤7,5, besonders bevorzugt pH ≤7,0 und insbesondere bevorzugt pH ≤6,5.

Entsprechend Gleichung (7) wird bei der Reaktion von OAS zu L-Cysteinsäure Essigsäure in stöchiometrischen Mengen freigesetzt, was im Verlauf der Reaktion zu einer Absenkung des pH-Werts im Ansatz führen kann. Da ein zu niedriger pH-Wert die Aktivität der OAS-Sulfhydrylase beeinträchtigt, sollte bevorzugt ein zu starkes Absinken des pH-Werts verhindert werden. Dies kann passiv durch einen geeigneten hochkonzentrierten Puffer im Ansatz erfolgen oder erfindungsgemäß aktiv durch eine Mess- und Regeleinheit bewerkstelligt werden. Erfindungsgemäß ist die aktive pH-Kontrolle durch eine Mess- und Regeleinheit, wie in Beispiel 5 offenbart, die bei Abweichung des pH-Wertes vom Sollwert durch Zudosierung einer Lauge oder Säure den gewünschten pH-Wert wieder einstellt (sog. pH-Stat Methode).

Biotransformation 1 kann in diskontinuierlicher oder kontinuierlicher Weise betrieben werden. Im diskontinuierlichen Betrieb (Batch Betrieb) werden dem Ansatz im Verlauf der Reaktion alle Reaktanden zugeführt und nach Beendigung der Reaktion der Ansatz aufgearbeitet. Im kontinuierlichen Betrieb wird während der Reaktion permanent OAS, CysM-Enzym und ein Salz der schwefligen Säure zudosiert und gleichzeitig dem Ansatz eine Lösung enthaltend das Produkt L-Cysteinsäure entnommen. Es wird ein Fließgleichgewicht eingestellt, bei dem die Reaktanden so zudosiert werden, dass sie während der Verweilzeit im Reaktionsgefäß zum Produkt L-Cysteinsäure abreagieren können. Ein Verfahren zur kontinuierlichen Produktion unnatürlicher Aminosäuren ist z.B. in EP 1 247 869 B1 (Wacker) offenbart. Bevorzugt handelt es sich bei Biotransformation 1 um ein diskontinuierliches Verfahren.

**Biotransformation 2:**

Das Verfahren zur Herstellung von Taurin umfasst die Herstellung von L-Cysteinsäure in Biotransformation 1 gefolgt von der Decarboxylierung von L-Cysteinsäure zu Taurin in Biotransformation 2.

Die Decarboxylierung von L-Cysteinsäure zu Taurin kann chemisch oder, erfindungsgemäß enzymatisch katalysiert, in einer Biotransformation erfolgen. Bekannt ist die nicht als nachhaltig anzusehende thermische Decarboxylierung bei hohen Temperaturen unter Metallkatalyse, die allerdings darunter leidet, dass sie energieintensiv ist und ein hoher Anteil an Nebenprodukten entsteht.

Bevorzugt ist das Verfahren dadurch gekennzeichnet, dass zur Decarboxylierung von L-Cysteinsäure zu Taurin ein Enzym aus der Klasse der L-Cysteinsulfinsäure-Decarboxylasen (CSAD, EC 4.1.1.29), der Aspartat-1-Decarboxylasen (EC 4.1.1.11) oder Glutamat-Decarboxylasen (EC 4.1.1.15) eingesetzt wird.

Zur erfindungsgemäßen, enzymatisch katalysierten Decarboxylierung von L-Cysteinsäure zu Taurin besonders bevorzugt sind Enzyme aus der Klasse der L-Cysteinsulfinsäure-Decarboxylasen (CSAD, EC 4.1.1.29). CSAD-Enzyme sind dafür bekannt, dass sie entsprechend Gleichung (3) L-Cysteinsulfinsäure zu Hypotaurin decarboxylieren. In unterschiedlichem Ausmaß sind diese Enzyme in der Lage, entsprechend Gleichung (5) auch L-Cysteinsäure als Substrat zu Taurin zu decarboxylieren. Wie in den Beispielen 7 bis 11 offenbart, ist z.B. das CSADcc-Enzym aus *Cyprinus carpio* (Karpfen) dazu geeignet, L-Cysteinsäure zu Taurin zu decarboxylieren.

Enzyme der Klasse EC 4.1.1.29 (CSAD) findet man hauptsächlich in Metazoen (vielzelligen Tieren), darunter in Säugetieren. Auch in Einzellern sind Enzyme mit CSAD-Aktivität zu finden wie in Algen z.B. aus der Gattung *Synechoccocus,* außerdem auch in Bakterien oder Pilzen. Bevorzugt ist das erfindungsgemäße Verfahren zur Herstellung von Taurin dadurch gekennzeichnet, dass CSAD-Enzyme der Klasse EC 4.1.1.29 aus Säugetieren, ausgewählt aus Mensch (*Homo sapiens*), Rind (*Bos taurus*), Ratte (Rattus norvegicus) oder Maus (Mus musculus) sowie aus Fischen wie z.B. aus Karpfen (*Cyprinus carpio*) verwendet werden, besonders bevorzugt Enzyme aus dem Menschen (*Homo sapiens*), der Ratte (*Rattus norvegicus*) oder dem Karpfen (*Cyprinus carpio*).

Insbesondere bevorzugt ist das CSAD-Enzym aus dem Karpfen (CSADcc, *Cyprinus carpio*). In den Beispielen verwendet wird ein CSADcc-Enzym, abgeleitet aus der Proteinsequenz des Wt-CSAD-Enzyms aus *Cyprinus carpio,* mit einer DNS-Sequenz der cds wie in SEQ ID NO: 1, nt 31 - 1530 offenbart und als CSADcc-cds bezeichnet, kodierend für ein Protein mit der Aminosäuresequenz in SEQ ID NO: 2 und bezeichnet als CSADcc. Die der Wt-CSADcc-Aminosäuresequenz zugrundeliegende DNS-Sequenz ist zugänglich in der NCBI (National Center for Biotechnology Information) Datenbank unter der Genbank Sequence ID: AB220585.1 (cds: nt 82 - 1584). Aus der korrespondierenden Wt-CSADcc-Aminosäuresequenz wurde eine für die Expression in *E. coli* codon-optimierte CSADcc-cds DNS-Sequenz abgeleitet (SEQ ID NO: 1, nt 31 - 1530), die für die identische Aminosäuresequenz codiert. Für die Codon-Optimierung stehen öffentlich zugängliche Software Programme zur Verfügung, wie z.B. die in Beispiel 6 verwendete Eurofins Genomics GENEius Software. Die DNS aus SEQ ID NO: 1 wurde in bekannter Weise synthetisch hergestellt, wie von Dienstleistern wie Eurofins Genomics angeboten.

Insbesondere bevorzugt handelt es sich bei der L-Cysteinsulfinsäure-Decarboxylase um SEQ ID NO: 2 oder eine zu dieser Sequenz homologe Sequenz.

Unter homologen Sequenzen ist zu verstehen, dass die DNS- oder Aminosäuresequenzen zu mindestens 80%, bevorzugt zu mindestens 90% und besonders bevorzugt zu mindestens 95% identisch sind, wobei jede Änderung in der homologen Sequenz ausgewählt ist aus Insertion, Addition, Deletion und Substitution einer oder mehrerer Nukleotide oder Aminosäuren.

Der Grad der DNA-Identität wird durch das Programm "nucleotide blast", zu finden auf der Seite http://blast.ncbi.nlm.nih.gov/, bestimmt, welches auf dem blastn-Algorithmus basiert. Als Algorithmus-Parameter für ein Alignment zweier oder mehrerer Nukleotidsequenzen wurden die voreingestellten Parameter genutzt. Die voreingestellten generellen Parameter sind: Max target sequences = 100; Short queries = "Automatically adjust parameters for short input sequences"; Expect Threshold = 10; Word size = 28; Automatically adjust parameters for short input sequences = 0. Die entsprechenden voreingestellten Scoring Parameter sind: Match/Mismatch Scores = 1,-2; Gap Costs = Linear.

Für den Vergleich von Proteinsequenzen wird das Programm "protein blast", auf der Seite http://blast.ncbi.nlm.nih.gov/, genutzt. Dieses Programm greift auf den blastp-Algorithmus zurück. Als Algorithmus-Parameter für ein Alignment zweier oder mehrerer Proteinsequenzen wurden die voreingestellten Parameter genutzt. Die voreingestellten generellen Parameter sind: Max target sequences = 100; Short queries = "Automatically adjust parameters for short input sequences"; Expect Threshold = 10; Word size = 3; Automatically adjust parameters for short input sequences = 0. Die voreingestellten Scoring Parameter sind: Matrix = BLOSUM62; Gap Costs = Existence: 11 Extension: 1; Compositional adjustments = Conditional compositional score matrix adjustment.

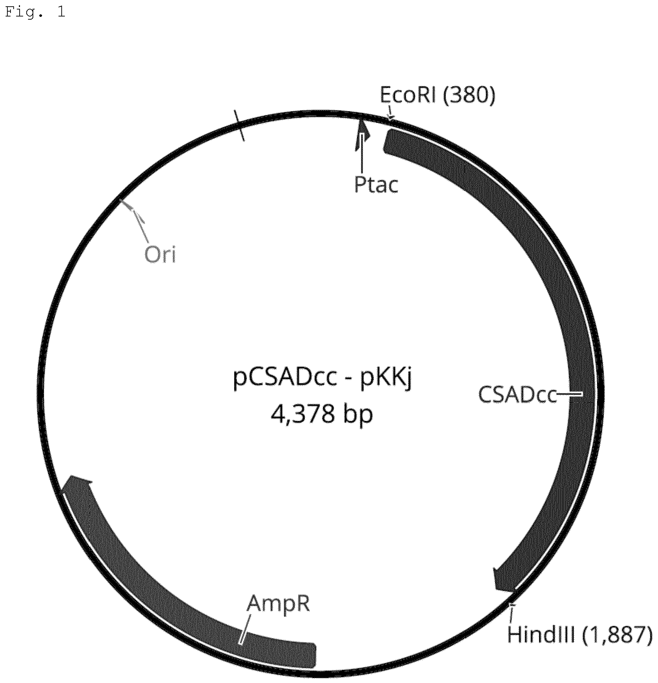

Bevorzugt ist das Verfahren dadurch gekennzeichnet, dass die L-Cysteinsulfinsäure-Decarboxylase aus fermentativer Herstellung stammt. Eine entsprechende rekombinante Produktion des CSADcc-Enzyms in einem *E. coli* Produktionsstamm ist in Beispiel 6 offenbart. Die CSADcc-cds wird dazu in bekannter Weise in einen Expressionsvektor, z.B. den Vektor pKKj, kloniert und das Genkonstrukt pCSADcc-pKKj hergestellt (Fig. 1). Zur Herstellung eines Produktionsstammes wird das Genkonstrukt pCSADcc-pKKj in ebenfalls bekannter Weise in einen *E. coli* Wirtsstamm, z.B. den Stamm *E. coli* JM105, transformiert und der resultierende Produktionsstamm *E. coli* JM105 x pCSADcc-pKKj in ebenfalls bekannter Weise zur Herstellung des CSADcc-Enzyms verwendet. Die Herstellung des CSADcc-Enzyms kann dabei für Laborzwecke im Schüttelkolbenmaßstab erfolgen oder durch Fermentation (Beispiel 6).

CSAD-Enzyme enthalten Pyridoxalphosphat (PLP, CAS Nr. 54-47-7) als Cofaktor. Die Supplementierung des Anzuchtmediums oder auch von Biotransformationsansätzen zur Umwandlung von L-Cysteinsäure zu Taurin mit PLP bietet daher eine Möglichkeit zur Verfahrensverbesserung. Da PLP zur Familie der B6-Vitamine gehört, eignet sich auch die Supplementierung mit anderen Vertretern der Vitamin B6-Familie wie z.B. Pyridoxin (CAS Nr. 65-23-6), Pyridoxal (CAS Nr. 66-72-8) oder Pyridoxamin (CAS Nr. 85-87-0) zur Verfahrensverbesserung. Bevorzugt ist das Biotransformationsverfahren zur Umwandlung von L-Cysteinsäure zu Taurin dadurch gekennzeichnet, dass es in Gegenwart von ≥20 mg/L, besonders bevorzugt ≥10 mg/L und insbesondere bevorzugt ≥4 mg/L PLP durchgeführt wird.

Das durch Anzucht im Schüttelkolben oder durch Fermentation gewonnene CSAD-Enzym, bevorzugt CSADcc, kann im erfindungsgemäßen Verfahren entweder als nicht weiter aufgearbeitete Fermenterbrühe eingesetzt werden oder aber auch als Zellsuspension nach Reisolierung der Zellen durch z.B. Zentrifugation und Resuspendierung der Fermenterzellen, z.B. in einem Puffer (resuspendierte Fermenterzellen). Weiterhin kann das CSAD-Enzym, bevorzugt CSADcc, in Form eines Zellhomogenats nach mechanischem Aufschluss der resuspendierten Fermenterzellen oder in Form chemisch permeabilisierter Zellen (z.B. durch Chloroform) eingesetzt werden oder aber auch als Zellextrakt nach Abtrennung partikulärer Bestandteile aus dem Zellhomogenat oder auch als z.B. chromatographisch gereinigtes Enzym.

Bevorzugt ist die Verwendung des CSAD-Enzyms (CSADcc), als nicht weiter aufgearbeitete Fermenterbrühe, als resuspendierte Fermenterzellen oder aber als Zellhomogenat nach mechanischem Aufschluss der resuspendierten Fermenterzellen. Besonders bevorzugt ist die Verwendung des CSAD-Enzyms als resuspendierte Fermenterzellen oder als Zellhomogenat nach mechanischem Aufschluss der resuspendierten Fermenterzellen. Insbesondere bevorzugt ist die Verwendung des CSAD-Enzyms in Form resuspendierter Fermenterzellen.

Die Biotransformation von L-Cysteinsäure zu Taurin durch das CSAD-Enzym, bevorzugt CSADcc, wird bevorzugt unter pH- und Temperaturbedingungen durchgeführt, die eine effiziente Decarboxylierung von L-Cysteinsäure zu Taurin ermöglichen. Der bevorzugte pH-Bereich, bei dem die Biotransformation 2 durchgeführt wird, liegt zwischen pH 5,0 und 9,0, besonders bevorzugt zwischen pH 6,0 und 8,5 und insbesondere bevorzugt zwischen pH 6,5 und 8,0.

Biotransformation 2 wird bei einer Temperatur bevorzugt <70°C, bevorzugt <60°C, besonders bevorzugt <50°C und insbesondere bevorzugt <40°C durchgeführt.

Die molare Ausbeute an Taurin aus der Biotransformation von L-Cysteinsäure in Biotransformation 2 beträgt bevorzugt mindestens 60 %, besonders bevorzugt mindestens 80% und insbesondere bevorzugt mindestens 90 %.

Die Biotransformation zur Herstellung von Taurin aus L-Cysteinsäure kann in diskontinuierlicher oder kontinuierlicher Weise betrieben werden. Im diskontinuierlichen Betrieb (Batch Betrieb) werden dem Ansatz im Verlauf der Reaktion alle Reaktanden zugeführt und nach Beendigung der Reaktion der Ansatz aufgearbeitet. Im kontinuierlichen Betrieb wird das CSAD-Enzym als stationäre Phase vorgelegt, z.B. in einem Membranreaktor oder an einen Träger immobilisiert und das Substrat L-Cysteinsäure als mobile Phase zudosiert. Die Kontaktzeit der mobilen Phase mit der stationären Phase wird dabei so eingestellt, dass das Substrat L-Cysteinsäure zum Produkt Taurin abreagieren kann. Bevorzugt ist der diskontinuierliche (Batch) Betrieb.

Das bevorzugte Verfahren zur Herstellung von Taurin aus OAS, umfassend Biotransformation 1 und Biotransformation 2, ist dadurch charakterisiert, dass es folgende Schritte umfasst:

a) OAS wird durch Fermentation hergestellt,
b) die Enzyme aus der Klasse der OAS-Sulfhydrylasen (EC 4.2.99.8) CysM und der Klasse der Cysteinsulfinsäure Decarboxylasen (EC 4.1.1.29), wie z.B. CSADcc, werden durch Fermentation hergestellt,
c) OAS und schweflige Säure, bzw. ein Salz der schwefligen Säure reagieren unter enzymatischer Katalyse der OAS-Sulfhydrylase aus Punkt b zu L-Cysteinsäure und
d) L-Cysteinsäure aus Punkt c wird durch das CSAD-Enzyms aus Punkt b zu Taurin decarboxyliert.

Insbesondere bevorzugt ist das Verfahren, umfassend die Schritte a, b, c und d, dadurch gekennzeichnet, dass in Biotransformation 1 OAS mit $Na_2SO_3$, bzw. dem Salz seines Anhydrids $Na_2S_2O_5$, in einer CysM-katalysierten Reaktion zu L-Cysteinsäure umgesetzt wird und in Biotransformation 2 die L-Cysteinsäure aus Biotransformation 1 in einer CSADkatalysierten Biotransformation nach Gleichung (5) zu Taurin decarboxyliert wird. Diese Verfahrensschritte sind in den Beispielen 7 bis 10 offenbart. OAS der Biotransformation 1 kann dazu synthetisch oder durch Anzucht eines Produktionsstammes hergestellt werden, wie in Beispiel 1 der vorliegenden Erfindung beschrieben. Bevorzugt ist die

Herstellung von OAS durch Anzucht eines Produktionsstammes. Das CysM-Enzym der Biotransformation 1 kann aus der Fermentation eines Produktionsstammes stammen, wie in Beispiel 2 beschrieben. Das in Biotransformation 2 verwendete CSAD-Enzym kann aus der Anzucht eines Produktionsstammes, bevorzugt das CSADcc-Enzym aus der Anzucht des Stammes *E. coli* JM105 x pCSADcc-pKKj, stammen, wie in Beispiel 6 beschrieben. L-Cysteinsäure aus Biotransformation 1 kann dabei ohne weitere Aufarbeitung in Biotransformation 2 eingesetzt werden, wie in den Beispielen 8 bis 10 beschrieben oder aber nach vorheriger Aufarbeitung. Dazu sind dem Fachmann verschiedene Methoden bekannt, wie z.B. Filtration, Zentrifugation, Extraktion, Adsorption, Ionenaustauscher-Chromatographie, Präzipitation, Kristallisation. Bevorzugt ist die Verwendung von L-Cysteinsäure aus Biotransformation 1 ohne weitere Aufarbeitung in Biotransformation 2.

Das Verfahren zur Herstellung von Taurin kombiniert in einfacher und effizienter Weise die enzymatische Herstellung von L-Cysteinsäure aus OAS und einem Salz der schwefligen Säure, auch bezeichnet als Sulfit, nach Gleichung (7) (Biotransformation 1) mit der enzymatischen Decarboxylierung von L-Cysteinsäure zu Taurin, nach Gleichung (5) (Biotransformation 2). Das bedeutet, dass die vorliegende Erfindung ein zweistufiges Biotransformationsverfahren aus der Kombination von Biotransformation 1 mit Biotransformation 2 zur Herstellung von Taurin aus OAS umfasst.

Bevorzugt laufen die Verfahrensschritte (Biotransformation 1 und Biotransformation 2) zur Herstellung von Taurin sequenziell, d.h. nacheinander ab. Wenn das Verfahren in der bereits beschriebenen bevorzugten Ausführungsform die Schritte a-d umfasst, ist es in einer alternativ bevorzugten Ausführungsform dadurch gekennzeichnet, dass alle Verfahrensschritte in einem Reaktionsansatz stattfinden. Wenn alle Verfahrensschritte in einem Reaktionsansatz stattfinden, spricht man auch von einem Eintopf-Verfahren bzw. einer Eintopfreaktion. Dieses hat den Vorteil, dass bereits alle Reaktanden zur Taurinherstellung im Reaktionsansatz enthalten sind oder einfach zudosiert werden können. Das Verfahren in einem Reaktionsansatz durchzuführen ist insbesondere wirtschaftlich gesehen von Interesse.

So offenbart Beispiel 11 der vorliegenden Erfindung, dass die Verfahrensschritte in einem Ansatz ablaufen können (Eintopfverfahren), wobei OAS mit einem Sulfit (Salz der schwefligen Säure) in Gegenwart der Enzyme CysM und CSADcc zur Reaktion gebracht wird. Dabei entsteht in der ersten Reaktion L-Cysteinsäure nach Gleichung (7), welches "in situ" durch CSADcc zu Taurin decarboxyliert wird nach Gleichung (5).

Die Produktverteilung von L-Cysteinsäure und Taurin aus der Reaktion von OAS im Eintopfverfahren wird bestimmt durch die Aktivität von CysM im Verhältnis zu CSADcc. Bei ausreichender Dosierung von CSADcc kann die aus OAS gebildete L-Cysteinsäure quantitativ zu Taurin umgewandelt werden. Bevorzugt ist ein Verfahren, bei dem das eingesetzte OAS zu L-Cysteinsäure und Taurin umgewandelt wird. Die aufsummierte molare Ausbeute von L-Cysteinsäure und Taurin, bezogen auf die eingesetzte molare Menge OAS, beträgt bevorzugt mehr als 60 %, besonders bevorzugt mehr als 70 % und insbesondere bevorzugt mehr als 80 %. Bezogen auf die eingesetzte molare Menge OAS beträgt die molare Ausbeute von Taurin bevorzugt mehr als 25%, besonders bevorzugt mehr als 50% und insbesondere bevorzugt mehr als 80%.

Mit Bezug auf ein Eintopfverfahren ist es denkbar, dass die Expression der Gene für die OAS-Sulfhydrylase und die L-Cysteinsulfinsäure-Decarboxylase gemeinsam in einem Stamm erfolgen kann und die Zellen aus der Anzucht dieses Stammes in einer Biotransformation in Gegenwart eines Sulfits mit OAS zur Reaktion gebracht werden, wobei als Endprodukt Taurin entsteht.

Es ist es auch denkbar, dass im Sinne eines Metabolic Engineering Ansatzes die Expression der Gene für eine OAS-Sulfhydrylase und einer L-Cysteinsulfinsäure Decarboxylase im OAS Produktionsstamm erfolgt und Taurin durch Anzucht eines solchen Produktionsstammes in Gegenwart eines Sulfits (Salz der schwefligen Säure) produziert wird.

Die Figur zeigt das in den Beispielen verwendete Plasmid.

Fig. 1: pCSADcc-pKKj

**In der Figur verwendete Abkürzungen:**

| | |
|---|---|
| AmpR: | Gen, das Resistenz gegenüber Ampicillin verleiht (ß-Lactamase) |
| Ori: | Replikationsursprung |
| Ptac: | tac-Promotor |
| EcoRI: | Schnittstelle für das Restriktionsenzym EcoRI |
| HindIII: | Schnittstelle für das Restriktionsenzym HindIII |
| CSADcc: | CSAD (Cysteinsulfinsäure Decarboxylase) *C. carpio* cds |

Die Erfindung wird durch die folgenden Beispiele weiter erläutert:

**Beispiel 1: Herstellung von OAS**

Verwendet wurde der in EP 1 233 067 B1 (Wacker) offenbarte Stamm *E. coli* W3110/pACYC-cysEX-GAPDH-ORF306, hinterlegt nach Budapester Vertrag bei der DSMZ Deutsche Sammlung für Mikroorganismen und Zellkulturen GmbH (Braunschweig) unter der Nummer DSM 13495. OAS wurde wie in EP 1 233 067 B1 beschrieben durch Fermentation hergestellt. Am Ende der Fermentation wurde zur Stabilisierung von OAS ein pH-Wert von 4,5 mit 21 % (v/v) Phosphorsäure eingestellt. Die Zellen wurden durch 10 min Zentrifugation bei 4000 rpm (Heraeus Megafuge 1.0 R) abgetrennt. Der durch HPLC bestimmt Gehalt an OAS im Fermentationsüberstand betrug 15,3 g/L.

HPLC-Analytik von OAS, L-Cysteinsäure und Taurin:

Zur quantitativen Bestimmung der in den Beispielen analysierten Verbindungen wurde eine jeweils für OAS, L-Cysteinsäure und Taurin kalibrierte HPLC-Methode eingesetzt, wobei alle zur Kalibrierung verwendeten Referenzsubstanzen kommerziell erhältlich waren (Sigma-Aldrich). Verwendet wurde ein HPLC-Gerät der Fa. Agilent, Modell 1260 Infinity II, ausgerüstet mit einer aus der Analytik von Aminosäuren bekannten Vorsäulenderivatisierung mit o-Phtaldialdehyd (OPA-Derivatisierung) vom gleichen Hersteller. Zum Nachweis der OPA-derivatisierten Produkte OAS, L-Cysteinsäure und Taurin war das HPLC-Gerät mit einem Fluoreszenzdetektor ausgerüstet. Der Detektor war eingestellt auf eine Excitationswellenlänge von 330 nm und eine Emissionswellenlänge von 450 nm. Des Weiteren verwendet wurde eine Accucore™ aQ Säule der Fa. Thermo Scientific™, Länge 100 mm, innerer Durchmesser 4,6 mm, Partikelgröße 2,6 $\mu$m, im Säulenofen auf 40°C temperiert. Laufmittel A: 25 mM Na-Phosphat, pH 6,0. Laufmittel B: Methanol. Die Trennung erfolgte im Gradientenmodus: 0 - 25 min, 10% Laufmittel B auf 60% Laufmittel B, gefolgt von 2 min 60% Laufmittel B auf 100% Laufmittel B, gefolgt von weiteren 2 min auf 100% Laufmittel B, bei einer Flußrate von 0,5 ml/min. Retentionszeit von L-Cysteinsäure: 3,2 min. Retentionszeit von Taurin: 14,8 min. Retentionszeit von OAS: 17,0 min.

**Beispiel 2: Herstellung des Enzyms CysM**

Verwendet wurde der in EP 1 247 869 B1 (Wacker) offenbarte Stamm *E. coli* DH5$\alpha$/pFL145, hinterlegt nach Budapester Vertrag bei der DSMZ Deutsche Sammlung für Mikroorganismen und Zellkulturen GmbH (Brauschweig) unter der Nummer DSM 14088. CysM-Enzym wurde sowohl durch Anzucht im Schüttelkolben wie durch Fermentation hergestellt.

A) Anzucht im Schüttelkolben: Vom Stamm *E. coli* DH5$\alpha$/pFL145 wurde in LBamp-Medium (10 g/l Trypton (GIBCO™), 5 g/l Hefeextrakt (BD Biosciences), 5 g/l NaCl, 100 mg/L Ampicillin (Sigma-Aldrich)) eine Vorkultur hergestellt (Anzucht bei 37°C und 120 rpm über Nacht). 25 ml Vorkultur wurden als Inokulum einer Hauptkultur von 250 ml LBamp-Medium (1 L Erlenmeyerkolben mit Schikane) verwendet. Die Hauptkultur wurde bei 30°C und 110 rpm geschüttelt. Nach 4 h wurde eine Zelldichte $OD_{600}$ von 1,0/ml erreicht ($OD_{600}$: photometrische Bestimmung der

Zelldichte/ml Zellsuspension durch Bestimmung der Extinktion bei 600 nm; Genesys™ 10S UV-Vis Spektralphotometer der Fa. Thermo Scientific™). Dann wurde der Induktor Tetracyclin (Sigma-Aldrich, 3 mg/L Endkonzentration) zugegeben und die Anzucht für weitere 20 h bei 30°C und 110 rpm fortgesetzt. Nach Beendigung der Anzucht betrug die Zelldichte $OD_{600}$ 3/ml.

B) Die fermentative Herstellung von CysM mit dem Stamm *E. coli* DH5α/pFL145 ist in EP 1 247 869 B1 offenbart. Die Zellen aus der Fermentation wurden durch 10 min Zentrifugation bei 4000 rpm (Heraeus Megafuge 1.0 R) abgetrennt und in KPi6,5-Puffer (0,1 M K-Phosphat, pH 6,5) suspendiert, sodass die Zelldichte $OD_{600}$ 90/ml betrug.

Die Zellen aus der Schüttelkolbenanzucht oder Fermentation wurden für die weitere Verwendung durch Zentrifugation isoliert (10 min 15000 rpm, Sorvall Zentrifuge RC5C, ausgestattet mit einem SS34 Rotor). Für die im Folgenden beschriebene weitere Verwendung zur Herstellung eines Zellhomogenats wurde das Zellpellet in KPi6,5-Puffer als Zellsuspension resuspendiert. Zur Herstellung der Zellsuspension wurde so viel KPi6,5-Puffer verwendet, dass die Zelldichte $OD_{600}$ 30/ml betrug: beispielsweise wurden 50 ml Zellen aus der Schüttelkolbenanzucht mit einer $OD_{600}$ von 3/ml zentrifugiert und in 5 ml KPi6,5-Puffer resuspendiert (10-fache Konzentrierung) oder 1 ml Zellen aus der Fermentation mit einer $OD_{600}$ von 90/ml in 3 ml KPi6,5-Puffer resuspendiert (3-fache Verdünnung).

Auf diese Weise wurden die aus der Fermenterbrühe isolierten und resuspendierten Zellen des Stammes *E. coli* DH5α/pFL145, die im Folgenden als OAS-Sulfhydrylase CysM in das erfindungsgemäße Verfahren eingesetzt wurden, erzeugt.

Zur Herstellung eines Zellhomogenats wurde der Zellhomogenisator FastPrep-24™ 5G der Fa. MP Biomedicals verwendet. 1 ml Zellsuspension in KPi6,5-Puffer mit einer Zelldichte $OD_{600}$ 30/ml wurde in vom Hersteller vorgefertigten 1,5 ml Röhrchen mit Glaskugeln ("Lysing Matrix B") aufgeschlossen (3 x 20 sec bei einer Schüttelfrequenz von 6000 rpm mit jeweils 30 sec Pause zwischen den Intervallen). Das erhaltene Zellhomogenat wurde direkt als OAS-Sulfhydrylase (CysM-Enzym) in das erfindungsgemäße Verfahren eingesetzt oder zur Herstellung eines Zellextrakts verwendet.

Zur Herstellung eines Zellextrakts wurde das erhaltene Zellhomogenat zentrifugiert (10 min 15000 rpm, Sorvall Zentrifuge RC5C, ausgestattet mit einem SS34 Rotor) und der Überstand mit Zellextrakt benannt und als OAS-Sulfhydrylase (CysM-Enzym) in das erfindungsgemäße Verfahren eingesetzt oder zur Bestimmung der CysM-Enzymaktivität weiterverwendet.

Der Proteingehalt des Zellextrakts wurde mit einem Qubit 3.0 Fluorometer der Fa. Thermo Fisher Scientific unter Einsatz des "Qubit® Protein Assay Kits" nach Angaben des Herstellers bestimmt. Der Proteingehalt des Zellextrakts aus der Schüttelkolbenanzucht betrug 5,3 mg/ml. Der Proteingehalt des Zellextrakts aus der Fermentation betrug 4,0 mg/ml.

Die CysM-Enzymaktivität wurde bestimmt wie in EP 1 247 869 B1 (Wacker) beschrieben. Dazu wurde OAS (Sigma-Aldrich) in Gegenwart von $Na_2S$ und Zellextrakt aus der Anzucht des Stammes *E. coli* DH5α/pFL145 bei 37°C inkubiert. Der Testansatz (0,4 ml Endvolumen) in KPi6,5-Puffer enthielt 10 mM OAS (Zugabe aus einer 200 mM Stammlösung in 500 mM Natrium-Succinat-Puffer pH 5,5), 10 mM Natriumsulfid $Na_2S$ und 5 µl CysM enthaltenden Zellextrakt. Das bei der CysM-Reaktion entstandene Cystein wurde mit Ninhydrin (Sigma-Aldrich) nach Gaitonde (1967), Biochem. J. 104: 627-633, bestimmt. Die CysM-Enzymaktivität im Zellextrakt aus der Anzucht des Stammes *E. coli* DH5α/pFL145 im Schüttelkolben betrug 57,1 U/ml. Da die Zellen aus der Schüttelkolbenanzucht ($OD_{600}$ von 3/ml) zur Herstellung des Zellextrakts 10-fach konzentriert worden waren, betrug die Enzymaktivität in Zellen aus der Schüttelkolbenanzucht 5,7 U/ml. Die CysM-Enzymaktivität im Zellextrakt nach Fermentation des Stammes E. coli DH5α/pFL145 betrug 58,1 U/ml. Da die Zellen aus der Fermentation ($OD_{600}$ von 90/ml) zur Herstellung des Zellextrakts auf eine $OD_{600}$ von 30/ml verdünnt worden waren, betrug die Enzymaktivität in der konzentrierten ($OD_{600}$ von 90/ml) Zellsuspension der Fermenterzellen 174,4 U/ml.

Die spezifische CysM-Enzymaktivität des Zellextrakts aus der Anzucht des Stammes *E. coli* DH5α/pFL145 im Schüttelkolben betrug 10,8 U/mg Protein. Die spezifische CysM-Enzymaktivität des Zellextrakts nach Fermentation des Stammes *E. coli* DH5α/pFL145 betrug 14,5 U/mg. Unter der Annahme, dass bei der Herstellung des Zellextrakts die CysM-Aktivität vollständig aus den Zellen freigesetzt wurde, wurde die in den Zellextrakten bestimmte CysM-Enzymaktivität in den folgenden Beispielen der in CysM-Zellsuspensionen enthaltenen Enzymaktivität gleichgesetzt.

1 U/ml CysM Enzymaktivität ist definiert als Produktion von 1 µmol Cystein/min aus OAS und $Na_2S$ unter Testbedingungen in 1 ml Zellextrakt (Volumenaktivität). Die spezifische CysM Enzymaktivität in U/mg Protein wird erhalten durch Division der Volumenaktivität des Zellextrakts (U/ml) durch die Proteinkonzentration des Zellextrakts (mg/ml) und ist definiert als CysM Enzymaktivität in U bezogen auf 1 mg Protein im Zellextrakt.

**Beispiel 3: Herstellung von L-Cysteinsäure aus kommerziell erhältlichem OAS und $Na_2SO_3$ mit Hilfe von CysM hergestellt in Schüttelkolbenkultur**

Zwei Ansätze wurden parallel durchgeführt:

**Ansatz 1:** In einem 100 ml Erlenmeyerkolben wurden 8,25 ml NaPi6,5-Puffer (50 mM Na-Phosphat, pH 6,5) vorgelegt und nacheinander 1 ml einer 0,2 M Lösung von $Na_2SO_3$ in NaPi6,5-Puffer, 0,4 ml CysM Zellextrakt aus der Schüttelkolbenanzucht (aus Beispiel 2A) mit einer Aktivität von 57,1 U/ml (2,3 U/ml Endkonzentration im Ansatz) und 350 µl einer 0,2 M Lösung von OAS x HCl (Sigma-Aldrich) in 0,5 M Na-Succinat, pH 5,5 zugegeben. Das Ansatzvolumen betrug 10 ml.

**Ansatz 2:** Der Ansatz (Vergleichsansatz ohne $Na_2SO_3$) hatte die gleiche Zusammensetzung wie Ansatz 1. Anstelle der $Na_2SO_3$-Lösung erhielt Ansatz 2 1 ml NaPi6,5-Puffer.

Beide Ansätze wurden in einem Truhenschüttler (Infors) bei 37°C und 140 rpm inkubiert. Nach 1 h und 3 h wurden jeweils 1 ml der Ansätze zum Stop der Reaktion 5 min bei 80°C inkubiert, zentrifugiert und der Überstand durch HPLC analysiert. Die mittels HPLC nachgewiesene Menge an L-Cysteinsäure ist in Tab. 1 wiedergegeben.

**Tabelle 1:** Mittels HPLC nachgewiesene Menge an L-Cysteinsäure in Abhängigkeit von der Reaktionszeit, wobei kommerziell erhältliches OAS, $Na_2SO_3$ und ein CysM-haltiger Zellextrakt eingesetzt wurden.

| Zeit [h] | Ansatz 1 mit $Na_2SO_3$ L-Cysteinsäure [mg/L] | Ansatz 2 ohne $Na_2SO_3$ L-Cysteinsäure [mg/L] |
|---|---|---|
| 0 | 0,0 | 0,0 |
| 1 | 78,0 | 0,0 |
| 3 | 95,8 | 0,0 |

### Beispiel 4: Herstellung von L-Cysteinsäure aus OAS-haltigem Kulturüberstand der Fermentation und $Na_2SO_3$ mit Hilfe von CysM hergestellt in Schüttelkolbenkultur

In einem 100 ml Erlenmeyerkolben wurden 1 ml Zellkulturüberstand aus der Fermentation des Stammes *E. coli* W3110/pACYC-cysEX-GAPDH-ORF306 mit einem Gehalt an OAS von 15,3 g/L (aus Beispiel 1) vorgelegt und nacheinander 6 ml NaPi6,5-Puffer, 1 ml einer 1 M Lösung von $Na_2SO_3$ in NaPi6,5-Puffer und 2 ml CysM-Zellsuspension aus der Schüttelkolbenanzucht (aus Beispiel 2A, Zelldichte $OD_{600}$ 30/ml; 57,1 U/ml CysM-Enzymaktivität) zugegeben. Das Ansatzvolumen betrug 10 ml. Die CysM-Enzymaktivität im Ansatz betrug 11,4 U/ml. Der Ansatz wurde in einem Truhenschüttler (Infors) bei 37°C und 140 rpm inkubiert. Nach 2 h wurde 1 ml des Ansatzes 5 min bei 80°C inkubiert, zentrifugiert und der Überstand durch HPLC auf den Gehalt an OAS und L-Cysteinsäure analysiert. Der zeitliche Verlauf der Reaktion ist in Tab. 2 zusammengefasst.

**Tabelle 2:** Mittels HPLC nachgewiesene Menge an L-Cysteinsäure und OAS, wobei ein OAS-haltiger Zellkulturüberstand, $Na_2SO_3$ und eine CysM-haltige Zellsuspension eingesetzt wurden.

| Zeit [h] | OAS [mg/L] | L-Cysteinsäure [mg/L] |
|---|---|---|
| 0 | 1530,0 | 0,0 |
| 2 | 0,0 | 1473,3 |

### Beispiel 5: Präparative Herstellung von L-Cysteinsäure durch Biotransformation von OAS bei konstantem pH

Ein doppelwandiges 0,5 L thermostatisierbares Glasgefäß (Diehm) wurde über eine Schlauchverbindung an einen Thermostaten (Lauda) angeschlossen und auf 37°C temperiert. 50 ml CysM-haltige Zellsuspension in KPi6,5-Puffer ($OD_{600}$ 90/ml, 8720 U CysM-Enzymaktivität) aus der Fermentation des Stammes DH5α/pFL145 (aus Beispiel 2B) sowie 6,6 ml einer 400 g/L Lösung von $Na_2S_2O_5$ (13,9 mmol, Molekulargewicht 190,1 g/mol) in KPi6,5-Puffer wurden vorgelegt. In gelöster Form entsprach das 27,8 mmol NaHSO3 (1,78-fach molarer Überschuss zur später zudosierten OAS-Menge von 15,6 mmol). Der Ansatz wurde mit einem Magnetrührer gerührt. Der Ansatz wurde weiterhin mit einer pH-Elektrode (Mettler Toledo) ausgestattet, welche mit einer pH-Kontrolleinheit (Titrator TitroLine alpha, Schott) verbunden war, die nach den Vorgaben des Herstellers im pH-Stat Modus betrieben wurde. Unter pH-Stat Bedingungen wurde der pH im Reaktionsgefäß während der gesamten Laufzeit der Reaktion konstant beim eingestellten pH 6,5 gehalten durch Zudosierung von 2 M NaOH aus einer mit der Kontrolleinheit verbundenen Bürette. 150 ml OAS-haltiger Zellkulturüberstand (OAS-Gehalt 15,3 g/L, 2,3 g; 15,64 mmol) aus der Fermentation des Stammes *E. coli* W3110/pACYC-cysEX-GAPDH-ORF306 (Beispiel 1) wurde aus einer Vorlage über eine Pumpe (Watson Marlow Peristaltikpumpe 101U/R) mit einer Flußrate von 0,35 ml/min in den Ansatz zudosiert.

Die Reaktionsdauer betrug 19 h. Da der Ansatz in einem offenen Reaktionsgefäß durchgeführt wurde, betrug das Ansatzvolumen infolge Verdunstung nach Beendigung der Reaktion 185 ml. 0,5 h, 3 h und 19 h nach Start der Reaktion wurde je 1 ml Aliquot des Ansatzes entnommen und der Gehalt an L-Cysteinsäure durch HPLC analysiert. Der zeitliche Verlauf der Bildung von L-Cysteinsäure ist in Tab. 3 zusammengefasst. Nach 19 h Reaktionsdauer betrug L-Cysteinsäure Gehalt im Ansatz 12970 mg/L (76,65 mM), was bei einem Ansatzvolumen von 185 ml einer absoluten molaren Ausbeute von 14,18 mmol L-Cysteinsäure entsprach. Bezogen auf die eingesetzte Menge OAS von 15,64 mmol entsprach dies einer Ausbeute von 90,1 %.

**Tabelle 3:** Mittels HPLC nachgewiesene Menge an L-Cysteinsäure in Abhängigkeit von der Reaktionszeit, wobei ein OAS-haltiger Fermentationsüberstand, $NaHSO_3$ und eine Zellsuspension von CysM-haltigen Fermenterzellen eingesetzt wurden

| Zeit [h] | L-Cysteinsäure [mg/L] | L-Cysteinsäure [mM] |
|---|---|---|
| 0,5 | 758,0 | 4,47 |
| 3 | 4244,0 | 25,08 |
| 19 | 12970,0 | 76,65 |

**Beispiel 6: Rekombinante Produktion der CSADcc aus *Cyprinus carpio* (Karpfen) in *E. coli***

**Vektor pCSADcc-pKKj:**

Das cDNS-Gen (cDNS: komplementäre DNS, durch reverse Transkription aus der mRNS isoliert) der Cysteinsulfinsäure Decarboxylase (CSAD) aus *Cyprinus carpio* (Karpfen) wurde von Honjoh et al. (2010), Amino Acids 38: 1173-1183 isoliert und die DNS-Sequenz in der NCBI (National Center for Biotechnology Information) Datenbank unter der Genbank Sequence ID: AB220585.1 (cds: nt 82 - 1584) offenbart. Aus der korrespondierenden Aminosäuresequenz wurde eine für die Expression in *E. coli* codon-optimierte DNS-Sequenz abgeleitet (öffentlich zugängliche Eurofins Genomics GENEius Software) und synthetisch hergestellt (Eurofins Genomics). Die synthetisch hergestellte DNS hatte die in SEQ ID NO: 1 offenbarte Sequenz und enthielt die cds des Gens, im Folgenden als CSADcc-cds bezeichnet (SEQ ID NO: 1, nt 31 - 1530), kodierend für ein Protein mit der in SEQ ID NO: 2 offenbarten Aminosäuresequenz und bezeichnet als CSADcc. Für Klonierungszwecke enthielt die synthetisch hergestellte DNS am 5'-Ende eine EcoRI Schnittstelle (SEQ ID NO: 1, nt 25 bis 30) und am 3'-Ende eine HindIII Schnittstelle (SEQ ID NO: 1, nt 1532 bis 1537).

Zur Herstellung des zur rekombinanten Expression der CSADcc-cds geeigneten Vektors pCSADcc-pKKj (Fig. 1) wurde die synthetisch hergestellte DNS mit EcoRI und HindIII geschnitten und in bekannter Weise als EcoRI/HindIII-Fragment in den mit EcoRI und HindIII geschnittenen Vektor pKKj kloniert. Der Expressionsvektor pKKj, offenbart in EP 2 670 837 A1, (Wacker) ist ein Derivat des Expressionsvektors pKK223-3. Die DNS-Sequenz von pKK223-3 ist offenbart in der GenBank Gendatenbank unter der Zugangsnummer M77749.1. Aus dem 4,6 kb Plasmid wurden ca. 1,7 kb entfernt (bp 262 - 1947 der in M77749.1 offenbarten DNS-Sequenz), wodurch der 2,9 kb Expressionsvektor pKKj entstand.

**Produktionsstamm *E. coli* JM105 x pCSADcc-pKKj:**

Zur Expression der CSADcc-cds in *E. coli* wurde der Vektor pCSADcc-pKKj in bekannter Weise in den Stamm *E. coli* K12 JM105 transformiert. Der Stamm *E. coli* K12 JM105 ist käuflich erhältlich unter der Stammnummer DSM 3949 bei der DSMZ Deutsche Sammlung von Mikroorganismen und Zellkulturen GmbH. Klone der Transformation wurden auf LBamp Platten selektiert. LBamp Platten enthielten 10 g/l Trypton (GIBCO™), 5 g/l Hefeextrakt (BD Biosciences), 5 g/l NaCl, 15 g/l Agar und 100 mg/L Ampicillin (Sigma-Aldrich). Ein Klon wurde ausgewählt für die Schüttelkolbenanzucht und Fermentation. Der CSADcc produzierende Stamm erhielt die Bezeichnung *E. coli* JM105 x pCSADcc-pKKj. Die Expression der CSADcc-cds erfolgte in *E. coli* JM105 x pCSADcc-pKKj in bekannter Weise unter Kontrolle des mit der CSADcc-cds funktionell verknüpften, IPTGinduzierbaren tac-Promotors (IPTG: Isopropyl-β-Thiogalactosid, Sigma-Aldrich).

**Anzucht im Schüttelkolben:**

Vom Stamm *E. coli* JM105 x pCSADcc-pKKj wurde in LBamp-Medium eine Vorkultur hergestellt (Anzucht bei 37°C und 120 rpm über Nacht, Infors Truhenschüttler).

Zwei ml Vorkultur wurden als Inokulum einer Hauptkultur von 100 ml SM3-Medium (1 L Erlenmeyerkolben), supplementiert mit 15 g/L Glucose, 5 mg/L Pyridoxalphosphat (PLP, Sigma-Aldrich) und 100 mg/L Ampicillin, verwendet. Die Hauptkultur wurde in einem Truhenschüttler (Infors) bei 30°C und 140 rpm geschüttelt. Nach 4 h Inkubationsdauer wurde eine Zelldichte $OD_{600}$ von 2,0 erreicht. Dann wurde der Induktor IPTG (Sigma-Aldrich, 0,4 mM Endkonzentration) zugegeben und die Anzucht für weitere 20 h in einem Truhenschüttler (Infors) bei 30°C und 140 rpm fortgesetzt.

Zusammensetzung des SM3-Mediums: 12 g/L $K_2HPO_4$, 3 g/L $KH_2PO_4$, 5 g/L $(NH_4)_2SO_4$, 0,3 g/L $MgSO_4$ x 7

$H_2O$, 0,015 g/L $CaCl_2$ x 2 $H_2O$, 0,002 g/L $FeSO_4$ x 7 $H_2O$, 1 g/L $Na_3$Citrat x 2 $H_2O$, 0,1 g/L NaCl; 5 g/L Pepton (Oxoid); 2,5 g/L Hefeextrakt (BD Biosciences); 0,005 g/L Vitamin B1; 1 ml/L Spurenelementlösung. Zusammensetzung der Spurenelementlösung: 0,15 g/L $Na_2MoO_4$ x 2 $H_2O$, 2,5 g/L $H_3BO_3$, 0,7 g/L $CoCl_2$ x 6 $H_2O$, 0,25 g/L $CuSO_4$ x 5 $H_2O$, 1,6 g/L $MnCl_2$ x 4 $H_2O$, 0,3 g/L $ZnSO_4$ x 7 $H_2O$.

Die Zellen aus der Schüttelkolbenanzucht wurden durch Zentrifugation isoliert. Zur Herstellung einer Zellsuspension wurde das Zellpellet aus 50 ml Schüttelkolbenanzucht in 2 ml 50 mM Na-Phosphat, pH 7,0 (NaPi7,0-Puffer) aufgenommen. Die Zellsuspension wurde entweder direkt für Biotransformationsversuche oder zur Herstellung eines Zellhomogenats verwendet.

Zur Herstellung eines Zellhomogenats wurde der Zellhomogenisator FastPrep-24TM 5G der Fa. MP Biomedicals verwendet. 2 x 1 ml Zellsuspension wurden in vom Hersteller vorgefertigten 1,5 ml Röhrchen mit Glaskugeln ("Lysing Matrix B") aufgeschlossen (3 x 20 sec bei einer Schüttelfrequenz von 6000 rpm mit jeweils 30 sec Pause zwischen den Intervallen). Das erhaltene Zellhomogenat (2 ml Volumen) wurde ohne weitere Aufarbeitung für die Biotransformation von L-Cysteinsäure zu Taurin verwendet.

**Anzucht durch Fermentation:**

Für die Fermentation verwendet wurde der Produktionsstamm *E. coli* JM105 x pCSADcc-pKKj. Die Fermentation wurde in einem Biostat B Fermenter (2 L Arbeitsvolumen) der Firma Sartorius BBI Systems GmbH durchgeführt.

Schüttelkolben Vorkultur:

100 ml LBamp-Medium in einem 1 L Erlenmeyerkolben wurde von einer Agar-Platte mit dem Stamm JM105 x pCSADcc-pKKj beimpft und auf einem Inkubationsschüttler (Infors) 7 - 8 h bei 30°C und einer Drehzahl von 120 rpm bis zu einer Zelldichte $OD_{600}$ von 2/ml - 4/ml inkubiert.

Vorfermenter:

1,5 L FM2-Medium, supplementiert mit 40 g/L Glucose und 100 mg/L Ampicillin, wurden mit 21,3 ml der Schüttelkolben-Vorkultur beimpft. Die Fermentationsbedingungen waren: Temperatur 30°C; pH-Wert konstant 7,0 (automatische Korrektur mit 25% $NH_4OH$ und 6,8 N $H_3PO_4$); Schaumbekämpfung durch automatische Zudosierung von 4 % v/v Struktol J673 in $H_2O$ (Schill & Seilacher); Rührerdrehzahl 450 - 1300 rpm; Belüftung mit über einen Sterilfilter entkeimter Druckluft konstant 1,7 vvm (vvm: Eintrag von Druckluft in den Fermentationsansatz angegeben in Liter Druckluft je Liter Fermentationsvolumen pro Minute); $pO_2 \geq 50\%$. Die Regulierung des Sauerstoffpartialdruckes $pO_2$ erfolgte über die Rührgeschwindigkeit. Nach einer Fermentationsdauer von 16 h wurde eine Zelldichte $OD_{600}$ von 45/ml erreicht.

Produktionsfermenter:

1,35 L FM2-Medium, pH 7,0, supplementiert mit 20 g/L Glucose, 0,36 g/L Pyridoxin (Vitamin B6, Sigma-Aldrich) und 100 mg/L Ampicillin, wurden mit 150 ml Vorfermenter-Kultur beimpft. Die Fermentationsbedingungen waren: Temperatur 30°C; pH-Wert konstant 7,0 (automatische Korrektur mit 25% $NH_4OH$ und 6,8 N $H_3PO_4$); Schaumbekämpfung durch automatische Zudosierung von 4 % v/v Struktol J673 in $H_2O$ (Schill & Seilacher); Rührerdrehzahl 450 - 1300 rpm; Belüftung konstant 1,7 vvm; $pO_2 \geq 50\%$. Die Regulierung des Sauerstoffpartialdruckes $pO_2$ erfolgte über die Rührgeschwindigkeit. Die Fermentationsdauer betrug 30 h.

FM2-Medium: $(NH_4)_2SO_4$, 5 g/L; NaCl, 0,50 g/L; $FeSO_4$ x 7 $H_2O$, 0,075 g/L; $Na_3$-Citrat, 1 g/L, $MgSO_4$ x 7 $H_2O$, 0,30 g/L, $CaCl_2$ x 2 $H_2O$, 0,015 g/L, $KH_2PO_4$, 1,50 g/L, Vitamin B1 (Sigma-Aldrich), 0,005 g/L; Pepton (Oxoid), 5,00 g/L; Hefeextrakt (Oxoid), 2,50 g/L; Spurenelementlösung, 10 ml/L (entspricht der für die Schüttelkolbenanzucht verwendeten).

Der pH-Wert im Fermenter wurde zu Beginn durch Zupumpen einer 25 % $NH_4OH$-Lösung auf 7,0 eingestellt. Während der Fermentation wurde der pH-Wert durch automatische Korrektur mit 25 % $NH_4OH$, bzw. 6,8 N $H_3PO_4$ auf einem Wert von 7,0 gehalten. Zum Animpfen wurden 150 ml Vorfermenter-Kultur in das Fermentergefäß gepumpt. Das Anfangsvolumen betrug somit 1,5 L. Die Kulturen wurden zu Beginn mit 350 rpm gerührt und mit einer Belüftungsrate von 1,7 vvm begast. Unter diesen Startbedingungen war die Sauerstoff-Sonde vor der Inokulation auf 100 % Sättigung kalibriert worden.

Der Soll-Wert für die $O_2$-Sättigung ($pO_2$) während der Fermentation wurde auf 50 % eingestellt. Nach Absinken der $O_2$-Sättigung unter den Soll-Wert wurde eine Regulationskaskade gestartet, um die $O_2$-Sättigung wieder an den Soll-Wert heranzuführen. Dabei wurde die Rührgeschwindigkeit (bis auf max. 1.300 rpm) kontinuierlich gesteigert.

Die Fermentation wurde bei einer Temperatur von 30°C durchgeführt. Sobald der Glucose-Gehalt im Fermenter von anfänglich 20 g/L auf ca. 5 g/L abgesunken war, erfolgte die kontinuierliche Zudosierung einer 60 % (w/w) Glucose-Lösung. Die Fütterungsrate wurde so eingestellt, dass die Glucosekonzentration im Fermenter 2 g/L fortan nicht mehr überstieg. Die Glucose-Bestimmung erfolgte mit einem Glucoseanalysator der Firma YSI (Yellow Springs, Ohio, USA).

Nachdem die Zelldichte im Fermenter eine $OD_{600}$ von 50/ml erreicht hatte (8 h Fermentationsdauer), wurde die Expression des CSADcc-Gens durch einmalige Zugabe des Induktors IPTG (0,2 mM Endkonzentration) gestartet. 22 h nach Induktion, entsprechend einer gesamten Fermentationsdauer von 30 h, wurde die Fermentation gestoppt. Die Zelldichte $OD_{600}$ betrug zu diesem Zeitpunkt 164/ml. 1 L der Fermenterbrühe wurde zentrifugiert (10 min 15000 rpm, Sorvall Zentrifuge RC5C, ausgestattet mit einem SS34 Rotor), der Fermentationsüberstand verworfen, die Zellen in 1 L NaPi7,0-Puffer resuspendiert und für die weitere Verwendung in 50 ml Aliquots bei -20°C gelagert.

**Beispiel 7: Herstellung von Taurin aus kommerziell erhältlicher L-Cysteinsäure durch Biotransformation**

In einem 100 ml Erlenmeyerkolben wurden 12 mg L-Cysteinsäure x $H_2O$ (Sigma-Aldrich) eingewogen und in 9,7 ml NaPi7,0-Puffer gelöst. Die Reaktion wurde gestartet durch Zugabe von 0,3 ml Zellhomogenat aus der Schüttelkolbenanzucht von JM105 x pCSADcc-pKKj (Beispiel 6). Das Ansatzvolumen betrug 10 ml. Die molare Konzentration der L-Cysteinsäure x $H_2O$ betrug 6,41 mM (Molekulargewicht L-Cysteinsäure x $H_2O$: 187,2 g/mol). Der Ansatz wurde in einem Truhenschüttler (Infors) bei 37°C und 140 rpm inkubiert. Nach 3 h wurde 1 ml des Ansatzes 5 min 80°C inkubiert, zentrifugiert und der Überstand durch HPLC analysiert. Die eingesetzte L-Cysteinsäure war vollständig verbraucht. Die gebildete Menge Taurin betrug 789,4 mg/L, entsprechend einem molaren Gehalt von 6,31 mM (Molekulargewicht Taurin: 125,1 g/mol). Entsprechend betrug die molare Ausbeute des aus 6,41 mM L-Cysteinsäure x $H_2O$ gebildeten Taurins 98,4 %.

**Beispiel 8: Herstellung von Taurin aus kommerziell erhältlichem OAS durch Biotransformation**

**Reaktion 1:** Herstellung von L-Cysteinsäure aus OAS:

In einem 100 ml Erlenmeyerkolben wurden 6,6 ml KPi6,5-Puffer vorgelegt und in schneller Folge 0,4 ml einer 0,2 M Stocklösung von OAS x HCl (Sigma-Aldrich), gelöst in 0,5 M Na-Succinat, pH 5,5, 1 ml 1 M $Na_2SO_3$ in KPi6,5-Puffer und 2 ml Zellsuspension von cysM-Zellen aus der Schüttelkolbenanzucht (aus Beispiel 2A; CysM Enzymaktivität 57,1 U/ml) zugegeben. Das Ansatzvolumen betrug 10 ml. Die Dosierung des CysM-Enzyms im Ansatz betrug 11,4 U/ml. Die molare Konzentration von OAS x HCl betrug 8,00 mM (1,47 g/L; OAS x HCl Molekulargewicht: 183,6 g/mol). Der Ansatz wurde in einem Truhenschüttler (Infors) bei 37°C und 140 rpm inkubiert. Nach 3 h wurde 1 ml des Ansatzes 5 min 80°C inkubiert, zentrifugiert und der Überstand durch HPLC analysiert. Das eingesetzte OAS war vollständig verbraucht. Die gebildete Menge L-Cysteinsäure betrug 1350,4 mg/L, entsprechend einem molaren Gehalt von 7,98 mM (Molekulargewicht L-Cysteinsäure: 169,2 g/mol). Die molare Ausbeute der aus 8,00 mM OAS x HCl gebildeten L-Cysteinsäure betrug 99,7 %.

**Reaktion 2:** Herstellung von Taurin aus der in Reaktion 1 synthetisierten L-Cysteinsäure:

In einem 100 ml Erlenmeyerkolben wurden 9 ml des Ansatzes aus Reaktion 1 vorgelegt, der pH mit 1 M KOH auf pH 7,0 eingestellt und 1 ml Zellhomogenat aus der Schüttelkolbenanzucht des Stammes JM105 x pCSADcc-pKKj zugegeben (Beispiel 6). Das Ansatzvolumen betrug 10 ml. Ausgehend von einem L-Cysteinsäure Gehalt von 1350,4 mg/L aus der Reaktion 1 betrug der L-Cysteinsäure Gehalt zu Beginn der Reaktion 2 1215,4 mg/L (7,18 mM bei einem Molekulargewicht von 169,2 für L-Cysteinsäure). Der Ansatz wurde in einem Truhenschüttler (Infors) bei 37°C und 140 rpm inkubiert. Nach 3 h wurde 1 ml des Ansatzes 5 min bei 80°C inkubiert, zentrifugiert und der Überstand durch HPLC analysiert. Die eingesetzte L-Cysteinsäure war vollständig verbraucht. Der Gehalt an Taurin betrug 825,7 mg/L (6,60 mM bei einem Molekulargewicht für Taurin von 125,1 g/mol). Die molare Ausbeute des aus 7,18 mM L-Cysteinsäure gebildeten Taurin betrug 91,9 %.

**Beispiel 9: Herstellung von Taurin durch CSADcckatalysierte Umsetzung von L-Cysteinsäure aus der Reaktion von fermentativ hergestelltem OAS mit $NaHSO_3$ und fermentativ hergestelltem CysM-Enzym**

In einem 100 ml Erlenmeyerkolben wurden 7 ml NaPi7,0-Puffer mit 5 mg/L PLP, 1 ml des Ansatzes aus Beispiel 5 mit einem Gehalt von L-Cysteinsäure von 12970 mg/L und 2 ml CSADcc-Zellen in NaPi7,0-Puffer (Beispiel 6) gemischt. Das Ansatzvolumen betrug 10 ml. Der L-Cysteinsäure Gehalt im Ansatz betrug 1297 mg/L, entsprechend einem molaren Gehalt von 7,67 mM (Molekulargewicht L-Cysteinsäure: 169,2 g/mol). Der Ansatz wurde in einem Truhenschüttler (Infors) bei 37°C und 140 rpm inkubiert. Nach 4 h wurde 1 ml des Ansatzes 5 min 80°C inkubiert, zentrifugiert und der Überstand durch HPLC analysiert. Die eingesetzte L-Cysteinsäure war vollständig verbraucht. Die gebildete Menge Taurin betrug 925,3 mg/L, entsprechend einem molaren Gehalt von 7,40 mM (Molekulargewicht Taurin: 125,1 g/mol). Die molare Ausbeute des aus 7,67 mM L-Cysteinsäure gebildeten Taurins betrug 96,4 %.

**Beispiel 10: Präparative Herstellung von Taurin**

**Biotransformation 1:**

Ein doppelwandiges 0,5 L thermostatisierbares Glasgefäß (Diehm) wurde über eine Schlauchverbindung an einen Thermostaten (Lauda) angeschlossen und auf 37°C temperiert. 50 ml CysM-haltige Zellsuspension in KPi6,5-Puffer (OD$_{600}$ 90/ml, 8720 U CysM-Enzymaktivität) aus der Fermentation des Stammes DH5α/pFL145 (aus Beispiel 2) sowie 20 ml einer 400 g/L Lösung von Na$_2$S$_2$O$_5$ (42,1 mmol, Molekulargewicht 190,1 g/mol) in KPi6,5-Puffer wurden vorgelegt. In gelöster Form entsprach das 84,2 mmol NaHSO3 (1,63-fach molarer Überschuss zur später zudosierten OAS-Menge von 51,7 mmol). Der Ansatz wurde mit einem Magnetrührer gerührt. Der Ansatz wurde weiterhin mit einer pH-Elektrode (Mettler Toledo) ausgestattet, welche mit einer pH-Kontrolleinheit (Titrator TitroLine alpha, Schott) verbunden war, die nach den Vorgaben des Herstellers im pH-Stat Modus betrieben wurde. Unter pH-Stat Bedingungen wurde der pH im Reaktionsgefäß während der gesamten Laufzeit der Reaktion konstant beim eingestellten pH 6,5 gehalten durch Zudosierung von 2 M NaOH aus einer mit der Kontrolleinheit verbundenen Bürette. 400 ml OAS-haltiger Fermentationsüberstand (OAS-Gehalt 19,1 g/L, 7,6 g; 51,7 mmol) aus der Fermentation des Stammes *E. coli* W3110/pACYC-cysEX-GAPDH-ORF306 (Beispiel 1) wurde aus einer Vorlage über eine Pumpe (Watson Marlow Peristaltikpumpe 101U/R) mit einer Flußrate von 0,2 ml/min in den Ansatz zudosiert. Die gesamte Reaktionsdauer betrug 46 h. Das Ansatzvolumen betrug nach Beendigung der Reaktion 500 ml. 1h, 3h, 20h, 28h und 46h nach Start der Reaktion wurde je 1 ml Aliquots des Ansatzes entnommen, 5 min bei 80°C inkubiert, zentrifugiert und der Überstand auf den Gehalt an L-Cysteinsäure durch HPLC analysiert. Der zeitliche Verlauf der Bildung von L-Cysteinsäure ist in Tab. 4 zusammengefasst. Nach 46 h Reaktionsdauer betrug L-Cysteinsäure Gehalt im Ansatz 15370 mg/L (90,8 mM), was bei einem Ansatzvolumen von 500 ml einer absoluten molaren Ausbeute von 45,4 mmol L-Cysteinsäure entsprach. Bezogen auf die eingesetzte Menge OAS von 51,7 mmol entsprach dies einer Ausbeute von 87,8 %.

**Tabelle 4:** Mittels HPLC nachgewiesene Menge an L-Cysteinsäure in Abhängigkeit von der Reaktionszeit, wobei ein OAS-haltiger Fermentationsüberstand, NaHSO$_3$ und eine Zellsuspension von CysM-haltigen Fermenterzellen eingesetzt wurden

| Zeit [h] | L-Cysteinsäure [mg/L] | L-Cysteinsäure [mM] |
|---|---|---|
| 1 | 709,0 | 4,2 |
| 3 | 1427,0 | 8,4 |
| 20 | 9814,0 | 58,0 |
| 28 | 10560,0 | 62,4 |
| 46 | 15370,0 | 90,8 |

**Biotransformation 2:**

Ein doppelwandiges 0,3 L thermostatisierbares Glasgefäß (Diehm) wurde über eine Schlauchverbindung an einen Thermostaten (Lauda) angeschlossen und auf 37°C temperiert. 100 ml L-Cysteinsäure-haltige Biotransformation 1 wurden mit 2,5 M NaOH auf pH 7,0 eingestellt. Weiter wurden 1 ml 1 M DTE (Dithioerythrol, Sigma-Aldrich), gelöst in H$_2$O, 1 ml 500 mg/L PLP (4 mg/L Endkonzentration), gelöst in NaPi7,0-Puffer und 20 ml CSADcc-haltige, in NaPi7,0-Puffer resuspendierte Fermenterzellen zugegeben. Der Ansatz wurde mit einem Magnetrührer gerührt. Das Ansatzvolumen betrug 122 ml. Zu Beginn der Reaktion und dann nach 2h, 4h, 6h und 24h wurden je 1 ml Aliquots des Ansatzes entnommen, 5 min bei 80°C inkubiert, zentrifugiert und der Überstand auf den Gehalt an L-Cysteinsäure und Taurin durch HPLC analysiert. Das Ergebnis ist in Tab. 5 zusammengefasst. Bezogen auf die molare eingesetzte Menge L-Cysteinsäure im Ansatz von 74,4 mM wurde eine Taurin-Ausbeute (8,8 g/L, 70,2 mM) von 94,3 % erzielt.

**Tabelle 5:** Mittels HPLC nachgewiesene Menge an L-Cysteinsäure und Taurin in Abhängigkeit von der Reaktionszeit, wobei ein L-Cystein-haltiger Fermentationsüberstand aus Biotransformation 1 und eine Zellsuspension von CSADcc-haltigen Fermenterzellen eingesetzt wurden

| Zeit [h] | L-Cysteinsäure [mg/L] | L-Cysteinsäure [MM] | Taurin [mg/L] | Taurin [mM] |
|---|---|---|---|---|
| 0 | 12592,5 | 74,4 | 0,0 | 0, 0 |
| 2 | 11821,1 | 69,88 | 1792,2 | 14,3 |
| 4 | 9106,7 | 53,83 | 2737,1 | 21,9 |

(fortgesetzt)

| Zeit [h] | L-Cysteinsäure [mg/L] | L-Cysteinsäure [MM] | Taurin [mg/L] | Taurin [mM] |
|---|---|---|---|---|
| 6 | 5159,6 | 30,5 | 4023,9 | 32,2 |
| 24 | 321,2 | 1,9 | 8783,6 | 70,2 |

**Beispiel 11: Herstellung von Taurin aus OAS in einer "Eintopfreaktion"**

Der Reaktionsansatz war zusammengesetzt aus 3 ml KPi6,5-Puffer, 2 ml OAS-haltigem Fermentationsüberstand aus der Fermentation des Stammes *E. coli* W3110/pACYC-cysEX-GAPDH-ORF306 (Beispiel 1), 1 ml 1 M Na$_2$SO$_3$ in KPi6,5-Puffer, 2 ml Zellsuspension CysM-haltiger Zellen aus der Fermentation des Stammes DH5α/pFL145 (Beispiel 2B) und 2 ml Zellsuspension CSADcc-haltiger Zellen aus der Schüttelkolbenanzucht des Stammes JM105 x pCSADcc-pKKj (Beispiel 6). Das Ansatzvolumen betrug 10 ml. Die OAS-Konzentration im Ansatz betrug 3,1 g/L (20,80 mM). Die Na$_2$SO$_3$-Konzentration im Ansatz betrug 100 mM. Die CysM-Enzymaktivität im Ansatz betrug 34,9 U/ml.

Die Reaktion wurde bei pH 6,5 durchgeführt. Der Ansatz wurde in einem Truhenschüttler (Infors) bei 37°C und 140 rpm inkubiert. 24 h nach Beginn der Reaktion wurde 1 ml des Ansatzes 5 min bei 80°C inkubiert, zentrifugiert und der Überstand durch HPLC analysiert. Der Gehalt an L-Cysteinsäure betrug 1,3 g/L (7,68 mM, bei einem Molekulargewicht für L-Cysteinsäure von 169,2 g/mol). Der Gehalt an Taurin betrug 721 mg/L (5,76 mM, bei einem Molekulargewicht für Taurin von 125,1 g/mol). Die molare Ausbeute der aus 20,80 mM OAS gebildeten 7,68 mM L-Cysteinsäure betrug 36,9 %. Die molare Ausbeute der aus 20,80 mM OAS gebildeten 5,76 mM Taurin betrug 27,7 %. Insgesamt betrug die molare Ausbeute der aus OAS gebildeten L-Cysteinsäure und Taurin 64,6 %.

**Patentansprüche**

1. Verfahren zur Herstellung von Taurin aus O-Acetyl-L-Serin (OAS) mittels Biotransformation, wobei

    i) in einem 1. Verfahrensschritt (Biotransformation 1) L-Cysteinsäure aus O-Acetyl-L-Serin (OAS) mit einem Enzym ausgewählt aus der Klasse der OAS-Sulfhydrylasen (EC 4.2.99.8) in Gegenwart eines Salzes der schwefligen Säure hergestellt wird,

       wobei die Biotransformation unter aktiver pH-Kontrolle durchgeführt wird
       und anschließend

    ii) in einem 2. Verfahrensschritt (Biotransformation 2) L-Cysteinsäure zu Taurin decarboxyliert wird,
    wobei die OAS-Konzentration im Ansatz mindestens 10 g/L beträgt und
    wobei es sich bei der OAS-Sulfhydrylase um CysM handelt.

2. Verfahren gemäß Anspruch 1, **dadurch gekennzeichnet, dass** es sich bei der OAS-Sulfhydrylase um ein bakterielles Enzym handelt.

3. Verfahren gemäß einem oder mehreren der Ansprüche 1 oder 2, **dadurch gekennzeichnet, dass** es sich bei der OAS-Sulfhydrylase um CysM des Stammes *E. coli* handelt.

4. Verfahren gemäß einem oder mehreren der Ansprüche 1 bis 3, **dadurch gekennzeichnet, dass** die OAS-Sulfhydrylase aus fermentativer Herstellung stammt.

5. Verfahren gemäß einem oder mehreren der Ansprüche 1 bis 4 **dadurch gekennzeichnet, dass** die Konzentration des Salzes der schwefligen Säure mindestens in äquimolarer Konzentration zu OAS vorliegt.

6. Verfahren gemäß einem oder mehreren der Ansprüche 1 bis 5, **dadurch gekennzeichnet, dass** zur Decarboxylierung von L-Cysteinsäure zu Taurin ein Enzym aus der Klasse der L-Cysteinsulfinsäure-Decarboxylasen (EC 4.1.1.29), der Aspartat-1-Decarboxylasen (EC 4.1.1.11) oder Glutamat-Decarboxylasen (EC 4.1.1.15) eingesetzt wird.

7. Verfahren gemäß Anspruch 6, **dadurch gekennzeichnet, dass** es sich bei der L-Cysteinsulfinsäure-Decarboxylase um SEQ ID NO: 2 oder eine zu dieser Sequenz homologe Sequenz handelt.

8. Verfahren gemäß einem oder mehreren der Ansprüche 6 oder 7, **dadurch gekennzeichnet, dass** die L- Cysteinsulfinsäure-Decarboxylase aus fermentativer Herstellung stammt.

9. Verfahren gemäß einem oder mehreren der Ansprüche 1 bis 8, **dadurch gekennzeichnet, dass**

a) OAS durch Fermentation hergestellt wird,
b) die Enzyme aus der Klasse der OAS-Sulfhydrylasen (EC 4.2.99.8) und der Klasse der Cysteinsulfinsäure Decarboxylasen (EC 4.1.1.29) durch Fermentation hergestellt werden,
c) OAS und ein Salz der schwefligen Säure unter enzymatischer Katalyse der OAS-Sulfhydrylase aus Punkt b zu L-Cysteinsäure reagieren und
d) L-Cysteinsäure aus Punkt c durch das CSAD-Enzyms aus Punkt b zu Taurin decarboxyliert wird.

10. Verfahren gemäß Anspruch 9, **dadurch gekennzeichnet, dass** alle Verfahrensschritte in einem Reaktionsansatz stattfinden.

11. Verfahren gemäß einem oder mehreren der Ansprüche 1 bis 10, **dadurch gekennzeichnet, dass** die molare Ausbeute an Taurin aus der Biotransformation von L-Cysteinsäure in Biotransformation 2 bevorzugt mindestens 60 % beträgt.

**Claims**

1. Process for producing taurine from O-acetyl-L-serine (OAS) by means of biotransformation, wherein

i) in a 1st process step (biotransformation 1), L-cysteic acid is produced from O-acetyl-L-serine (OAS) using an enzyme selected from the class of OAS sulfhydrylases (EC 4.2.99.8) in the presence of a salt of sulfurous acid,

wherein the biotransformation is carried out under active pH control,
and then

ii) in a 2nd process step (biotransformation 2), L-cysteic acid is decarboxylated to taurine,
wherein the OAS concentration in the batch is at least 10 g/L and
wherein the OAS sulfhydrylase is CysM.

2. Process according to Claim 1, **characterized in that** the OAS sulfhydrylase is a bacterial enzyme.

3. Process according to one or both of Claims 1 and 2, **characterized in that** the OAS sulfhydrylase is CysM from the strain *E. coli.*

4. Process according to one or more of Claims 1 to 3, **characterized in that** the OAS sulfhydrylase stems from fermentative production.

5. Process according to one or more of Claims 1 to 4, **characterized in that** the concentration of the salt of sulfurous acid is at least in equimolar concentration to OAS.

6. Process according to one or more of Claims 1 to 5, **characterized in that** L-cysteic acid is decarboxylated to taurine using an enzyme from the class of L-cysteine sulfinic acid decarboxylases (EC 4.1.1.29), aspartate 1-decarboxylases (EC 4.1.1.11) or glutamate decarboxylases (EC 4.1.1.15).

7. Process according to Claim 6, **characterized in that** the L-cysteine sulfinic acid decarboxylase is SEQ ID NO: 2 or a sequence homologous to this sequence.

8. Process according to one or both of Claims 6 and 7, **characterized in that** the L-cysteine sulfinic acid decarboxylase stems from fermentative production.

9. Process according to one or more of Claims 1 to 8, **characterized in that**

a) OAS is produced by fermentation,

b) the enzymes from the class of OAS sulfhydrylases (EC 4.2.99.8) and the class of cysteine sulfinic acid decarboxylases (EC 4.1.1.29) are produced by fermentation,
c) OAS and a salt of sulfurous acid react to form L-cysteic acid under enzymatic catalysis by the OAS sulfhydrylase from point b, and
d) L-cysteic acid from point c is decarboxylated to taurine by the CSAD enzyme from point b.

10. Process according to Claim 9, **characterized in that** all the process steps take place in one reaction batch.

11. Process according to one or more of Claims 1 to 10, **characterized in that** the molar yield of taurine from the biotransformation of L-cysteic acid in biotransformation 2 is preferably at least 60%.

**Revendications**

1. Procédé de production de taurine à partir d'O-acétyl-L-sérine (OAS) par biotransformation, dans lequel

i) dans une 1ère étape de procédé (biotransformation 1) de l'acide L-cystéique est produit à partir d'O-acétyl-L-sérine (OAS) avec une enzyme choisie dans la classe des OAS-sulfhydrylases (EC 4.2.99.8) en présence d'un sel de l'acide sulfureux,

la biotransformation étant effectuée sous contrôle actif du pH
et ensuite

ii) dans une 2ème étape de procédé (biotransformation 2), l'acide L-cystéique est décarboxylé en taurine, la concentration d'OAS dans la préparation étant d'au moins 10 g/l, et l'OAS-sulfhydrylase consistant en la CysM.

2. Procédé selon la revendication 1, **caractérisé en ce que** l'OAS-sulfhydrylase consiste en une enzyme bactérienne.

3. Procédé selon une ou plusieurs des revendications 1 ou 2, **caractérisé en ce que** l'OAS-sulfhydrylase consiste en la CysM de la souche *E. coli.*

4. Procédé selon une ou plusieurs des revendications 1 à 3, **caractérisé en ce que** l'OAS-sulfhydrylase est issue d'une production par fermentation.

5. Procédé selon une ou plusieurs des revendications 1 à 4 **caractérisé en ce que** la concentration du sel de l'acide sulfureux est au moins en concentration équimolaire par rapport à l'OAS.

6. Procédé selon une ou plusieurs des revendications 1 à 5, **caractérisé en ce que**, pour la décarboxylation de l'acide L-cystéique en taurine, on utilise une enzyme de la classe des décarboxylases de l'acide L-cystéique-sulfinique (EC 4.1.1.29), des décarboxylases de l'aspartate 1 (EC 4.1.1.11) ou des décarboxylases de glutamate (EC 4.1.1.15).

7. Procédé selon la revendication 6, **caractérisé en ce que** la décarboxylase de l'acide L-cystéique-sulfinique consiste en la SEQ ID NO : 2 ou en une séquence homologue à cette séquence.

8. Procédé selon une ou plusieurs des revendications 6 ou 7, **caractérisé en ce que** la décarboxylase de l'acide L-cystéine-sulfinique est issue d'une production par fermentation.

9. Procédé selon une ou plusieurs des revendications 1 à 8, **caractérisé en ce que**

a) l'OAS est produite par fermentation,
b) les enzymes de la classe des OAS-sulfhydrylases (EC 4.2.99.8) et de la classe des décarboxylases de l'acide cystéine-sulfinique (EC 4.1.1.29) sont produites par fermentation,
c) l'OAS et un sel de l'acide sulfureux réagissent sous catalyse enzymatique de l'OAS-sulfhydrylase du point b pour former l'acide L-cystéique ; et
d) l'acide L-cystéique du point c est décarboxylé en taurine par l'enzyme CSAD du point b.

10. Procédé selon la revendication 9, **caractérisé en ce que** toutes les étapes du procédé ont lieu dans une préparation réactionnelle.

11. Procédé selon une ou plusieurs des revendications 1 à 10, **caractérisé en ce que** le rendement molaire en taurine de la biotransformation de l'acide L-cystéique dans la biotransformation 2 est de préférence d'au moins 60 %.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 17213142 A1, Ajinomoto
- US 9267148 B2
- US 20120222148 A1
- US 20180028474 A1
- US 20190085339 A
- WO 2017176277 A1
- WO 2019094051 A
- US 20190062757 A1

- EP 1191106 B1, Wacker
- EP 1247869 B1, Wacker
- EP 4441239 A1
- EP 1233067 B1, Wacker
- EP 1233067 A
- EP 2670837 A1, Wacker

**In der Beschreibung aufgeführte Nicht-Patentliteratur**

- *CHEMICAL ABSTRACTS*, 107-35-7
- **SALZE** ; **DAVIS**. *Aquaculture*, 2015, vol. 437, 215-229
- **RIPPS** ; **SHEN**. *Molecular Vision*, 2012, vol. 18, 2673-2686
- *CHEMICAL ABSTRACTS*, 207121-48-0
- *CHEMICAL ABSTRACTS*, 23537-25-9
- *CHEMICAL ABSTRACTS*, 300-84-5
- **HONJOH et al.** *Amino Acids*, 2010, vol. 38, 173-1183
- **TEVATIA et al.** *Algal Research*, 2019, vol. 40, 101491
- **JOO et al.** *J. Agric. Food Chem.*, 2018, vol. 66, 13454-13463
- **ONO et al.** *Free Radical Biology and Medicine*, 2017, vol. 106, 69-79

- **S. MAIER**. *Nature Biotechnology*, 2003, vol. 21, 422-427
- **TAI et al.** *Biochemistry*, 1995, vol. 34, 12311-12322
- **SIEPER et al.** *Rapid Commun. Mass Spectrom.*, 2006, vol. 20, 2521-2527
- *CHEMICAL ABSTRACTS*, 1637-71-4
- *CHEMICAL ABSTRACTS*, 54-47-7
- *CHEMICAL ABSTRACTS*, 65-23-6
- *CHEMICAL ABSTRACTS*, 66-72-8
- *CHEMICAL ABSTRACTS*, 85-87-0
- **GAITONDE**. *Biochem. J.*, 1967, vol. 104, 627-633
- **HONJOH et al.** *Amino Acids*, 2010, vol. 38, 1173-1183